# EUROPEAN PATENT APPLICATION

(11) **EP 1 630 174 A1**
(43) Date of publication of application: **01.03.2006**
(21) Application number: 04732817.4
(22) Date of filing: 13.05.2004
(51) Int. Cl.: C07K 14/705, C12N 15/12, C12N 1/15, C12N 1/19, C12N 1/21, C12N 5/00, C07K 16/28, C12P 21/02, C12Q 1/02, C12Q 1/68, A01K 67/027, G01N 33/15, G01N 33/50, G01N 33/53, G01N 33/566

(54) **LIVER X RECEPTOR ALPHA SPLICING MUTANT PROTEIN, GENE THEREOF AND UTILIZATION OF THE SAME**

(30) Priority: 14.05.2003 JP 2003135461
(71) Applicant: Sumitomo Chemical Company, Limited, Tokyo 104-8260 (JP)
(72) Inventor: FUJIMORI, Ko, 5670012 (JP); SAITO, Koichi, 6650847 (JP)
(74) Representative: Vossius & Partner
(86) International application number: PCT/JP2004/006814
(87) International publication number: WO 2004/101616

(57) **Abstract**

The present invention provides a liver X receptor α splicing mutant protein which is an isoform of a liver X receptor α, and which comprises at least an amino acid sequence encoded by exon 5 of a liver X receptor α gene, a gene thereof, and use thereof, and these can be utilized in development of methods and agents useful for preventing, diagnosing or treating diseases and abnormalities involved in inhibition of normal cholesterol metabolism by a normal liver X receptor α.

## Description

### TECHNICAL FIELD

The present invention relates to liver X receptor α splicing mutant proteins which are isoforms of liver X receptor α, genes thereof, and use thereof.

### BACKGROUND ART

Cholesterol is an important lipid *in vivo,* and is also a component constituting various lipids. Cholesterol is absorbed from intestinal tract through ingestion, or biosynthesized from acetyl-CoA in liver. Biosynthesized cholesterol is excreted by liver, re-absorbed in small intestine, transported to liver via blood, and re-utilized. In addition, in liver, a part of cholesterol is metabolized into bile acid. When a cholesterol level *in vivo* is increased due to any abnormality, it causes development of hyperlipemia or atherosclerosis derived from hypercholesterolemia. Mechanism of pathogenesis of these diseases derived from hypercholesterolemia has been not sufficiently elucidated. Currently, as an agent for treating diseases derived from hypercholesterolemia, cholesterol synthesis inhibitors(statin preparations) or fibrate agents are used. However, treatment with these agents is not necessarily sufficiently satisfactory in some cases.

Re-absorption and conversion into bile acid of cholesterol (cholesterol metabolism) are mediated by each particular transporter or enzyme. Further, it is known that a liver X receptor which is one of nuclear receptors is involved in regulating expression of a group of genes encoding these proteins.

As a liver X receptor, it has hitherto been reported that there are two kinds of subtypes (liver X receptor α, liver X receptor β) (see, for example, Peet et al., Curr.Opin.Genet.Dev. 8; 571-575, 1998). It is known that a liver X receptor α regulates transcription of a target gene by forming a heterodimer with a retinoid X receptor (RXR) which is one of nuclear receptors, and binding to a transcription regulatory region of the target gene. Also in nuclear receptors other than liver X receptor, subtypes and isoforms have been found out and, for example, in RAR, RXR and PPAR, three kinds of subtypes as well as isoforms in which proteins encoded by them are altered, have been found out (see, for example, Mangelsdorf and Evans, Cell, 83: 841-850, 1995). It is thought that isoforms of RAR and PPAR are generated by selective splicing and/or control of different promoters, and it is known that their roles and tissue specificity of expression are varied (see, for example, Takeyama et al., Biochem. Biophys. Res. Commun. 222; 395-400, 1996). Therefore, it is thought that subtypes and isoforms of nuclear receptors have different functions, respectively. Regarding a liver X receptor α carrying an important role in regulating cholesterol metabolism, if there is an isoform having a function to change normal cholesterol metabolism mediated by a normal liver X receptor α, such the isoform is important for elucidating relationship with disease symptom directly or indirectly associated with the function, and developing an agent for preventing or treating those diseases.

### DISCLOSURE OF THE INVENTION

The present invention provides liver X receptor α splicing mutant proteins which are novel isoforms of a liver X receptor α involved in inhibition of normal cholesterol metabolism with a normal liver X receptor α, genes thereof, and use thereof.

More specifically, the present invention provides:
1. a liver X receptor α splicing mutant protein which is an isoform of a liver X receptor α, and which comprises at least an amino acid sequence encoded by exon 5 of a liver X receptor α gene (hereinafter, referred to as splicing mutant protein of the present invention in some cases);
2. a liver X receptor α splicing mutant protein which is an isoform of a liver X receptor α, and which contains an amino acid sequence encoded by intron 5 of a liver X receptor α gene (hereinafter, referred to as splicing mutant protein 5A of the present invention in some cases);
3. the liver X receptor α splicing mutant protein according to the item 2, which comprises any of the following amino acid sequences:
   (1) the amino acid sequence of SEQ ID NO: 1,
   (2) an amino acid sequence which is substantially identical to the amino acid sequence of SEQ ID NO: 1, and
   (3) an amino acid sequence which has 95% or more amino acid ident ity with the amino acid sequence of SEQ ID NO: 1;
4. a liver X receptor α splicing mutant protein which is an isoform of a liver X receptor α, and which contains an amino acid sequence encoded by a portion of intron 6 of a liver X receptor α gene (hereinafter, referred to as splicing mutant protein 6A of the present invention in some cases);
5. the liver X receptor α splicing mutant protein according to the item 4, which comprises any of the following amino acid sequences:
   (1) the amino acid sequence of SEQ ID NO: 2,
   (2) an amino acid sequence which is substantially identical to the amino acid sequence of SEQ ID NO: 2, and
   (3) an amino acid sequence which has 95% ormore amino acid identity with the amino acid sequence of SEQ ID NO: 2;
6. the liver X receptor α splicing mutant protein according to any one of the items 1 to 5, which has weaker transcription activating ability as compared with ligand-dependent transcription activating ability of a normal liver X receptor α;
7. a polynucleotide comprising
   a nucleotide sequence encoding an amino acid sequence of the liver X receptor α splicing mutant protein as defined in the item 3, or
   a nucleotide sequence complementary to said nucleotide sequence;
8. a polynucleotide comprising
   the nucleotide sequence of SEQ ID NO: 3, or
   a nucleotide sequence complementary to said nucleotide sequence;
9. a polynucleotide comprising
   a nucleotide sequence encoding an amino acid sequence of the liver X receptor α splicing mutant protein as defined in the item 5, or
   a nucleotide sequence complementary to said nucleotide sequence;
10. a polynucleotide comprising
   the nucleotide sequence of SEQ ID NO: 4, or
   a nucleotide sequence complementary to said nucleotide sequence;
11. a polypeptide which is a partial polypeptide of the liver X receptor α splicing mutant protein as defined in the item 1, and which comprises an amino acid sequence that is not present in a normal liver X receptor α, within an amino acid sequence of the liver X receptor α splicing mutant protein as defined in the item 1 (hereinafter, referred to as polypeptide of the present invention in some cases);
12. the polypeptide according to the item 11, which comprises any of the following amino acid sequences:
   (1) the amino acid sequence of SEQ ID NO: 5, and
   (2) the amino acid sequence of SEQ ID NO: 6;
13. a polynucleotide comprising
   a nucleotide sequence encoding an amino acid sequence of a polypeptide as defined in the item 12, or
   a nucleotide sequence complementary to said nucleotide sequence;
14. a polynucleotide comprising
   the nucleotide sequence of SEQ ID NO: 7, or
   a nucleotide sequence complementary to said nucleotide sequence;
15. a polynucleotide comprising
   the nucleotide sequence of SEQ ID NO: 8, or
   a nucleotide sequence complementary to said nucleotide sequence;
16. a recombinant vector (hereinafter, referred to as vector of the present invention in some cases) containing
   a polynucleotide that comprises a nucleotide sequence encoding an amino acid sequence of the liver X receptor α splicing mutant protein as defined in any one of the items 1 to 6, or
   a polynucleotide as defined in any one of the items 7 to 10 (hereinafter, generally, referred to a splicing mutant protein gene of the present invention in some cases);
17. a transformant in which
   a polynucleotide comprising a nucleotide sequence encoding an amino acid sequence of the liver X receptor α splicing mutant protein as defined in any one of the items 1 to 6, or
   a polynucleotide as defined in any one of the items 7 to 10
   is introduced into a host cell (hereinafter, referred to as transformant of the present invention in some cases);
18. a process for producing a vector comprising a step of introducing a polynucleotide that comprises a nucleotide sequence encoding an amino acid sequence of the liver X receptor α splicing mutant protein as defined in any one of the items 1 to 6, or a polynucleotide as defined in any one of the items 7 to 10, into a vector capable of autonomously replicating in a host cell (hereinafter, referred to as a process for producing a vector of the present invention in some cases);
19. a process for producing a liver X receptor α splicing mutant protein comprising a step of culturing a transformant as defined in the item 17 to produce a liver X receptor α splicing mutant protein (hereinafter, referred to as a process for producing a splicing mutant protein of the present invention in some cases);
20. a gene-deficient animal which is deficient in the ability to express a polynucleotide comprising a nucleotide sequence encoding an amino acid sequence of a liver X receptor α splicing mutant protein as defined in any one of the items 1 to 6, or a polynucleotide as defined in any one of the items 7 to 10;
21. a recombinant vector containing a polynucleotide that comprises a nucleotide sequence encoding an amino acid sequence of a polypeptide as defined in any one of the items 11 to 12 (hereinafter, referred to as polypeptide gene of the present invention in some cases), or a polynucleotide as defined in any one of the items 13 to 15;
22. a transformant in which a polynucleotidecomprising a nucleotide sequence encoding an amino acid sequence of a polypeptide as defined in any one of the items 11 to 12, or a polynucleotide as defined in any one of the items 13 to 15 is introduced into a host cell ;
23. a method for screening a substance which acts on a liver X receptor α splicing mutant protein, comprising a step of contacting a test substance with a liver X receptor α splicing mutant protein, and a step of measuring a change in the liver X receptor α splicing mutant protein (hereinafter, referred to as screening method of the present invention in some cases);
24. the screening method according to the item 23, wherein the liver X receptor α splicing mutant protein is a liver X receptor α splicing mutant protein as defined in any one of the items 1 to 6;
25. a method for screening a substance which acts on a liver X receptor α splicing mutant protein, comprising a step of contacting a test substance with a cell producing a liver X receptor α splicing mutant protein, a step of measuring an amount of production of other protein which undergoes expression regulation by the liver X receptor α splicing mutant protein in the cell, and a step of assessing the property of acting of the test substance on the liver X receptor α splicing mutant protein based on the amount of production ;
26. the screening method according to the item 25, wherein the liver X receptor α splicing mutant protein is a liver X receptor α splicing mutant protein as defined in any one of the items 1 to 6;
27. a method for screening a substance which acts on a liver X receptor α splicing mutant protein, comprising a first step of contacting a cell producing a liver X receptor α splicing mutant protein with a ligand for the liver X receptor α splicing mutant protein, and measuring an amount of production of other protein which undergoes expression regulation by the liver X receptor α splicing mutant protein, a second step of contacting a cell producing the liver X receptor α splicing mutant protein with both of the ligand for the liver X receptor α splicing mutant protein and a test substance, and measuring an amount of production of other protein which undergoes expression regulation by the liver X receptor α splicing mutant protein, and a step of assessing of the properties of the test substance of antagonizing the ligand based on a difference in amounts of production of said other proteins measured in the first step and the second step ;
28. the screening method according to the item 27, wherein the liver X receptor α splicing mutant protein is a liver X receptor α splicing mutant protein as defined in any one of the items 1 to 6;
29. a substance which acts on a liver X receptor α splicing mutant protein, and which has been selected by the screening method as defined in any one of the items 23 to 28;
30. an antagonist to a substance as defined in the item 29, wherein said substance acts on a liver X receptor α splicing mutant protein;
31. an antibodywhich specifically recognizes a liver X receptor α splicing mutant protein as defined in any one of the items 1 to 6, or a part of the antibody;
32. a primer designed based on a polynucleotide as defined in any one of the items 7 to 10;
33. use of a DNA probe specific for a nucleotide sequence of a polynucleotide as defined in any one of the items 13 to 15, for gene therapy;
34. use of an RNA probe specific for a nucleotide sequence of a polynucleotide as defined in any one of the items 13 to 15, for gene therapy;
35. a method for detecting the presence or the absence of a liver X receptor α splicing mutant protein as defined in any one of items 1 to 6, comprising a first step of synthesizing complementary DNA (cDNA) from messenger RNA (mRNA) in a biological sample, a second step of amplifying a portion of the complementary DNA (cDNA) corresponding to a polynucleotide that comprises a nucleotide sequence encoding an amino acid sequence of a liver X receptor α splicing mutant protein as defined in any one of items 1 to 6 or a polypeptide as defined in any one of the items 11 to 12, or a polynucleotide as defined in any one of the items 13 to 15, and a third step of detecting the amplified portion;
36. the method according to the item 35, wherein the portion is amplified by the PCR method using, as a primer, one or a plurality of polynucleotides selected from polynucleotides comprising any of nucleotide sequences of SEQ ID NO: 9, SEQ ID NO: 10, SEQ ID NO: 11, SEQ ID NO: 12, SEQ ID NO: 13 and SEQ ID NO: 14 in a second step;
37. the method according to the item 35, wherein the polynucleotide comprising a nucleotide sequence encoding an amino acid sequence of a liver X receptor α splicing mutant protein as defined in any one of the items 1 to 6 or a polypeptide as defined in any one of items 11 to 12, or the polynucleotide as defined in any one of the items 13 to 16 is a polynucleotide comprising a nucleotide sequence of SEQ ID NO: 7 or 8;
38. a method for detecting the presence or the absence of a liver X receptor α splicing mutant protein as defined in any one of the items 1 to 6, comprising a step of detecting the liver X receptor α splicing mutant protein based on an antigen-antibody reaction using an antibody that can specifically detect a liver X receptor α splicing mutant protein as defined in any one of the items 1 to 6 or a polypeptide as defined in any one of the items 11 to 12;
39. a method for detecting the presence or the absence of a liver X receptor α splicing mutant protein as defined in any one of the items 1 to 6, comprising a first step of binding to a microtiter well a primary antibody which can specifically detect a liver X receptor α splicing mutant protein as defined in any one of the items 1 to 6 or a polypeptide as defined in any one of the items 11 to 12, a second step of binding to the primary antibody on the microtiter well a liver X receptor α splicing mutant protein as defined in any one of the items 1 to 6 in a biological sample, a third step of removing all biological samples which are in an unbound state in the microtiter well after the second step, a forth step of binding to the liver X receptor α splicing mutant protein bound to the primary antibody a labeled secondary antibody which can bind to an epitope different from those for the primary antibody in the liver X receptor α splicing mutant protein, a fifth step of removing all secondary antibodies which are in an unbound state in the microtiterwell after the forth step , and a sixth step of detecting the presence or the absence of the secondary antibody using a label on the secondary antibody as an index after the fifth step;
40. use of a liver X receptor α splicing mutant protein as a marker for investigating cholesterol metabolism abnormality;
41. the use according to the item 40, wherein the liver X receptor α splicing mutant protein is a liver X receptor α splicing mutant protein as defined in the item 2, 3, 4, 5 or 6, or a liver X receptor α splicing mutant protein which is an isoform of a liver X receptor α, and which is deficient in an amino acid sequence encoded by exon 5 of a liver X receptor α gene;
42. use of a liver X receptor α splicing mutant protein which is an isoform of a liver X receptor α, and which is deficient in an amino acid sequence encoded by exon 5 of a liver X receptor α gene, as a marker for investigating the presence of a cancer cell;
43. use of a polynucleotide comprising a nucleotide sequence encoding an amino acid sequence of a liver X receptor α splicing mutant protein, as a marker for investigating cholesterol metabolism abnormality;
44. the use according to the item 43, wherein the polynucleotide comprising a nucleotide sequence encoding an amino acid sequence of a liver X receptor α splicing mutant protein is a polynucleotide as defined in any one of the items 7 to 10 and 13 to 15; and
45. use of a polynucleotide comprising a nucleotide sequence encoding an amino acid sequence of a liver X receptor α splicing mutant protein, wherein said protein is an isoform of a liver X receptor α and is deficient in an amino acid sequence encoded by exon 5 of a liver X receptor α gene, as a marker for investigating the presence of a cancer cell; and the like.

### BRIEF DESCRIPTION OF THE DRAWINGS

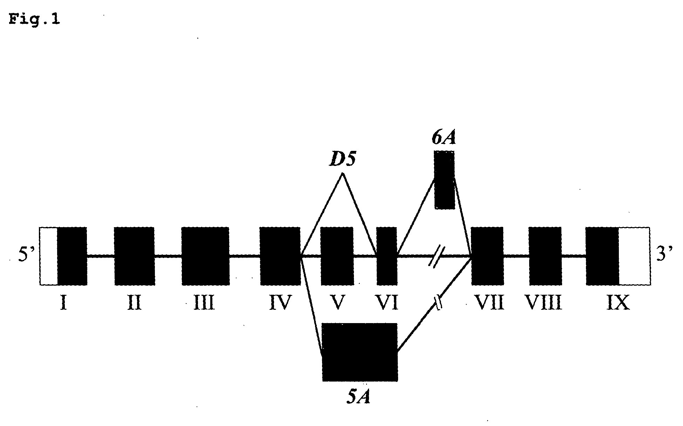

Fig. 1 is a view schematically showing structural domains of a normal liver X receptor α gene and three kinds of liver X receptor α splicing mutant protein genes. A translated region is indicated by black.

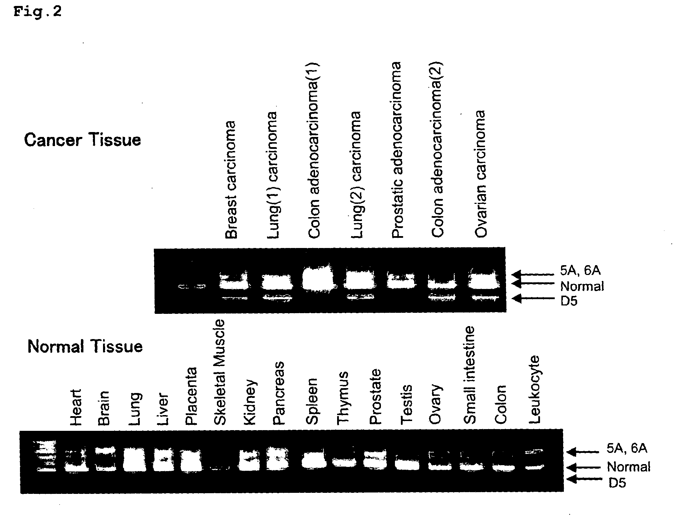

Fig. 2 is a view showing the results obtained by subjecting an aliquot of the PCR product amplified by PCR which was performed by using a human normal tissue or human cancer tissue-derived cDNA as a template and using primers (LXRαF and LXRαR) specific for a liver X receptor α, to electrophoresis of a 1% agarose gel containing ethidium bromide and, after electrophoresis, visualizing amplified signals under ultraviolet-ray irradiation. Breast carcinoma; Lung carcinoma; Colon adenocarcinoma; Prostatic adenocarcinoma; Ovarian carcinoma.

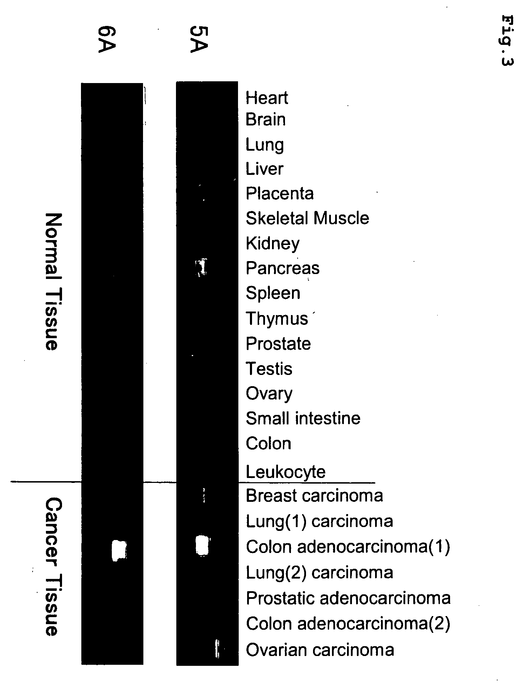

Fig. 3 is a view showing the PCR products amplified by PCR which was performed using a human normal tissue or human cancer tissue-derived cDNA as a template and using primer sets for specifically amplifying each of three kinds of liver X receptor α splicing mutant proteins. The figure at an upper row shows the PCR products amplified by PCR which was performed using a primer comprising the nucleotide sequence of SEQ ID NO: 9 and a primer comprising the nucleotide sequence of SEQ ID NO: 13, said primers being designed so as to specifically amplify the liver X receptor α splicing mutant protein 5A. The PCR product derived from hLXRα5A is a DNA fragment of 432 base pairs. The figure at a lower row shows the PCR products amplified by PCR which was performed using a primer comprising the nucleotide sequence of SEQ ID NO: 9 and a primer comprising the nucleotide sequence of SEQ ID NO: 14, said primers being designed so as to specifically amplify the liver X receptor α splicing mutant protein 6A. The PCR product derived from a liver X receptor α splicing mutant protein 6A is a DNA fragment of 429 base pairs. PCR products obtained by respective PCR reactions were determined for their nucleotide sequences after TA cloning, and it was confirmed that the intended products were amplified. Breast carcinoma; Lung carcinoma; Colon adenocarcinoma; Prostatic adenocarcinoma; Ovarian carcinoma.

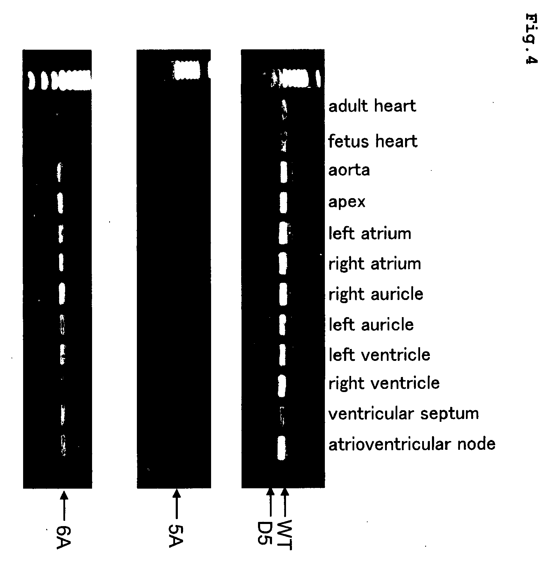

Fig. 4 is a view showing the results of investigation of expression of a normal liver X receptor α and three kinds of liver X receptor α splicing mutant proteins in circulatory tissues. The figure at an upper row is one example of investigation of expression of a normal liver X receptor α and the liver X receptor α splicing mutant protein D5. The PCR product derived from a liver X receptor α splicing mutant protein D5 is a DNA fragment of 416 base pairs. The figure at a middle row shows the products amplified by PCR which was performed using a primer comprising the nucleotide sequence of SEQ ID NO: 9 and a primer comprising the nucleotide sequence of SEQ ID NO: 13, said primers being designed so as to specifically amplify the liver X receptor α splicing mutant protein 5A. The PCR product derived from hLXRα5a is a DNA fragment having 432 base pairs. The figure at a lower row shows the products amplified by PCR which was performed using a primer comprising the nucleotide sequence of SEQ ID NO: 9 and a primer comprising the nucleotide sequence of SEQ ID NO: 14, said primers being designed so as to specifically amplify the liver X receptor α splicing mutant protein 6A. The PCR product derived from a liver X receptor α splicing mutant protein 6A is a DNA fragment of 429 base pairs. The PCR products obtained by respective PCR reactions were determined in their nucleotide sequences after TA cloning, and it was confirmed that the intended products were amplified.

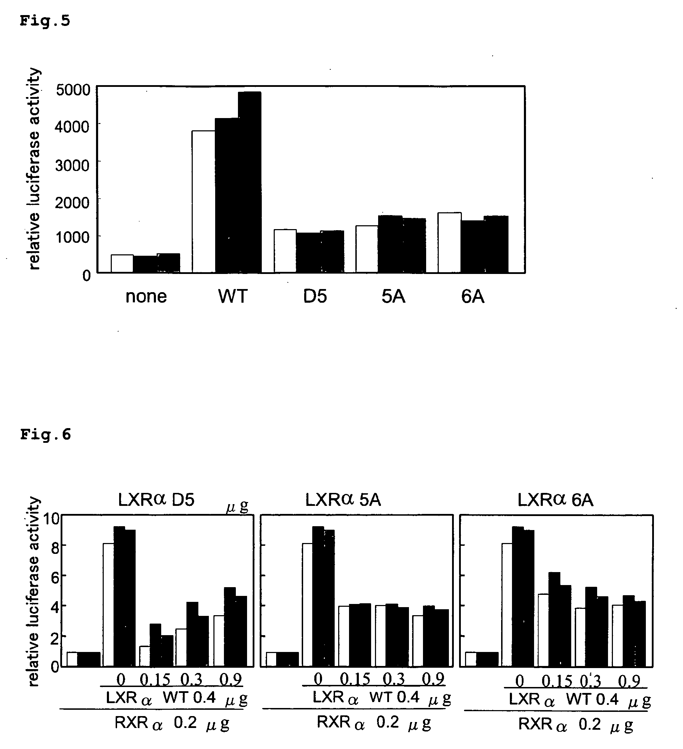

Fig. 5 is a view showing the results of assessment of the function of liver X receptor α splicing mutant proteins (D5, 5A and 6A) by a reporter assay in a transient expression system in HEK293 cells. The figure shows the results obtained by introducing TATA-LXREx4-luciferase vector and each of three kinds of liver X receptor α splicing mutant protein expression vectors into HEK293 cells, and measuring the transcription activating ability when various liver X receptor α ligands were added. A non-treated section is represented by white, a 1 µg/ml 22R-hydroxycholesterol-treated section is represented by black, and a 1 µg/ml 25-hydroxycholesterol-treated section is represented by gray. In addition, in each test section, 1 µM 9-cis retinoic acid is contained. Letting a value of luciferase activity in a control section (control) in which only a solvent was added to be 100%, a value of luciferase activity in each test section is shown as a relative value. WT means a normal liver X receptor α.
Fig. 6 is a view showing the results obtained by measuring transcription activating ability when a normal liver X receptor α (LXRαWT) expression vector and a liver X receptor α splicing mutant protein (LXRαD5, LXRα5A, LXRα6A) expression vector are present together in HEK293 cells. A non-treated section is represented by white, a 1 µg/ml 22R-hydroxy cholesterol-treated section is represented by black, and a 1 µg/ml 25-hydroxycholesterol-treated section is represented by gray.
In addition, in each test section, 1 µM 9-cis retinoic acid is contained. Letting a value of luciferase activity in a control section (control) to which only a solvent was added to be 100%, a value of luciferase activity in each test section is indicated as a relative value.

### BEST MODE FOR CARRYING OUT THE INVENTION

The present invention will be explained in detail below.

The present invention relates to liver X receptor α splicing mutant proteins which are isoforms of a liver X receptor α, genes thereof, and use thereof.

Among three kinds of liver X receptor α splicing mutant proteins isolated in the present invention, one is an isoform in which an amino acid sequence encoded by intron 5 is contained (splicing mutant protein 5A of the present invention), and other one is an isoform in which an amino acid sequence encoded by a portion of intron 6 is contained (splicing mutant protein 6A of the present invention). These amino acid sequences are newly found sequences. Further one is an isoform in which an amino acid sequence encoded by exon 5 is deficient (liver X receptor α splicing mutant protein D5). The amino acid sequence of this isoform was identical to the sequence already registered in public database (GenBank Accession No. BC008819).

The splicing mutant protein 5A of the present invention has a nucleotide sequence identical to that of normal type except that an amino acid sequence encoded by intron 5 is contained downstream of an amino acid residue corresponding to the carboxyl terminus of an amino acid sequence encoded by exon 5 of a normal liver X receptor α. In addition, the splicing mutant protein 6A of the present invention has a nucleotide sequence identical to that of normal type except that an amino acid sequence encoded by a partial region in intron 6 is contained downstream of an amino acid residue corresponding to the carboxyl terminus of an amino acid sequence encoded by exon 6. The liver X receptor α splicing mutant protein D5 has an amino acid sequence identical to that of normal type except that an amino acid sequence encoded by exon 5 is deleted.

The transcription activating ability depending on 22R-oxycholesterol that is one of ligands for a liver X receptor α, possessed by these three kinds of liver X receptor α splicing proteins is at an extremely weak or not recognized level as compared with the transcription activating ability possessed by a normal liver X receptor α. Further, when a normal liver X receptor α and the aforementioned three kinds of each liver X receptor α splicing mutant proteins are coexpressed, ligand-dependent transcription activation observed when only a normal liver X receptor α is expressed, is remarkably suppressed. From the above findings, it is suggested that the three kinds of liver X receptor α splicing mutant proteins are involved in inhibition of normal cholesterol metabolism by a normal liver X receptor α in a dominant negative manner *in vivo.*

The splicing mutant proteins of the present invention are isoforms of a liver X receptor α, and contain at least an amino acid sequence encoded by exon 5.

Representative examples include an isoform of a liver X receptor α in which an amino acid sequence encoded by intron 5 of a liver X receptor α gene is contained (i.e. splicing mutant protein 5A of the present invention), an isoform of a liver X receptor α in which an amino acid sequence encoded by a portion of intron 6 of a liver X receptor α gene is contained (i.e. splicing mutant protein 6A of the present invention) and the like.

Further, specifically, examples of the splicing mutant protein 5A of the present invention include a liver X receptor α isoform comprising any amino acid sequence of (1) the amino acid sequence represented by SEQ ID NO: 1, (2) an amino acid sequence which is substantially identical to the amino acid sequence of SEQ ID NO: 1, and (3) an amino acid sequence which has 95% or more amino acid identity with the amino acid sequence of SEQ ID NO: 1. In addition, examples of the splicing mutant protein 6A of the present invention include a liver X receptor α isoform comprising any amino acid sequence of (1) the amino acid sequence of SEQ ID NO: 2, (2) an amino acid sequence which is substantially identical to the amino acid sequence of SEQ ID NO: 2, and (3) an amino acid sequence which has 95% or more amino acid identity with the amino acid sequence of SEQ ID NO: 2.

Such the splicing mutant protein of the present invention has weaker transcription activating ability as compared with ligand-dependent transcription activating ability of a normal liver X receptor α.

Herein, regarding definition of "an amino acid sequence which is substantially identical to" , it is the well-known fact that, generally, in the case where an amino acid sequence of a protein having physiological activity is slightly changed, for example, even in the case where there is a change such as deletion, substitution and addition of one or a plurality of amino acids in the amino acid sequence, physiological activity of the protein is maintained in some cases. Therefore, "an amino acid sequence which is substantially identical to" referred in the present specification means that, as far as biological activity substantially equivalent to that of a particular amino acid sequence (i.e. amino acid sequence of SEQ ID NO: 1 or 2) is retained, a human liver X receptor α splicing mutant protein in which one or a plurality of amino acids in the amino acids sequence have been deleted, substituted or added is also included in the scope of the present invention. The number of amino acids to be altered in the foregoing is at least one residue, specifically, one or several (herein, "several" is around 2 to about 10) or more. Such the number of alterations may be in such the range that physiological activity of the protein is maintained. More specifically, it is a human liver X receptor α splicing mutant protein in which one or more and 20 or less, preferably one or more and 10 or less, further preferably one or more and 5 or less amino acids in the amino acid sequence of SEQ ID NO: 1 or 2 are deleted, substituted or added. Such the mutation may be, for example, a naturally occurring mutation resulting from processing which a protein undergoes in a cell, a difference in species of an organism, individuals, organs, tissues, or the like from which the protein is derived, or may be an artificial amino acid mutation (e.g. mutation in an amino acid present in an amino acid sequence of a protein produced by introducing a mutation into DNA encoding a natural protein by site-directed mutagenesis, mutagenic treatment or the like, followed by expressing it).

Such the mutant protein generated by deletion, substitution or addition of amino acids may contain a conservatively substituted amino acid sequence. This means that a particular amino acid residue may be substituted with a residue having physiochemical similarility (e.g. nature similar in hydrophobicity, charge, pK, stereo-structural feature and the like). Non-limiting examples of such the conservative substitution include substitution between aliphatic chain-containing amino acid residues, and substitution between polar groups, such as substitution in groups of (i) glycine, alanine; (ii) valine, isoleucine, leucine; (iii) aspartic acid, glutamic acid, asparagine, glutamine, (iv) serine, threonine; (v) lysine, arginine; (vi) phenylalanine, tyrosine.

A mutant protein generated by deletion, substitution or addition of an amino acid can be obtained by performing, for example, site-directed mutagenesis which is the known technique (e.g. Nelson and McClelland, Methods Enzymol, 216; 279 , 1992, a method of utilizing amber mutation (gapped-duplex method, Nucleic Acids Res., 12, 9441-9456, 1984), a method by PCR using a primer for introducing mutation) on a gene comprising a nucleotide sequence encoding an amino acid sequence thereof.

Site-directed mutagenesis can be performed by using a synthetic primer containing mutation to be introduced. That is, using the aforementioned synthetic oligonucleotide and a primer having a nucleotide sequence complementary to a nucleotide sequence thereof as primers, and employing a plasmid containing a gene of a normal liver X receptor α as a template, an amplification reaction is performed. Then, treatment with DpnI which is a methylation-sensitive restriction enzyme leaves only a newly generated DNA having the mutation. Using this reaction solution, Escherichia coli XLI-Blue strain is transformed, and spread on an ampicillin-containing LB agar medium. This is cultured at 37°C overnight, and a plasmid is isolated from the grown colony. Thereby, a plasmid containing a mutated DNA can be obtained. As a kit based on the aforementioned method, for example, QuickChange Site-Directed Mutagenesis Kit (manufactured by Stratagene Corporation) or the like is commercially available, and this may be utilized. Introduction of intended mutation can be confirmed by determining a nucleotide sequence thereof.

Further, examples of a method of performing deletion, substitution or addition of an amino acid sequence include a method of treating a gene with a mutagen, and a method of cleaving a gene with a restriction enzyme, followed by removing, adding or substituting a selected gene fragment, and further followed by ligating it, in addition to the aforementioned site-directed mutagenesis.

Herein, "normal liver X receptor α" means a liver X receptor α comprising an amino acid sequence which occurrs in nature most frequently in amino acid sequences of the receptor protein derived from an organism of the same species. Examples of a human-derived normal liver X receptor α include a liver X receptor α comprising an amino acid sequence registered in public database (GenBank Accession No. NM_005693).

"Amino acid identity", and "nucleotide identity" in the present invention refer to identity and homology of sequences between two proteins or two DNAs. The "identity" is determined by comparing two sequences aligned in the optimal state, over an entire region of sequences to be compared. Herein, proteins or DNAs to be compared may have addition or deletion (e.g. gap etc.) in the optimal alignment of two sequences. Such the identity can be calculated, for example, by producing an alignment using VectorNTI utilizing Clustal W algorithm (Nucleic Acid Res., 22(22): 4673-4680 (1994). The identity is measured using a sequence analyzing software, specifically, Vector NTI, GENETYX-MAC, or an analyzing tool provided by public databases. The public databases are generally available, for example, at a homepage address http://www.ddbj.nig.ac.jp.

"Amino acid identity" in the present invention is based on an amino acid sequence, and is preferably, for example, about 95% or more. "Nucleotide identity" is based on a nucleotide sequence, and is preferably, for example, about 95% or more.

The splicing mutant proteins of the present invention can be obtained by the conventional genetic engineering method such as a hybridization method and a PCR method.

For example, RNA is extracted from an animal tissue of a mammal such as human, monkey, rabbit, rat, mouse and the like or a cultured cell derived from such the animal according to the genetic engineering method described in such as Sambrook and Russell; Molecular Cloning 3^{rd} edition, Cold Spring Harbor Laboratory (2001), and a single-stranded cDNA is synthesized. Specifically, for example, a tissue such as liver is homogenized in a solution containing a protein denaturing agent such as guanidine thiocyanate and, further, chloroform or the like is added to the homogenate to denature proteins. After the denatured proteins are removed by centrifugation, total RNA is extracted from the recovered supernatant fraction using phenol, chloroform or the like. Examples of a commercially available kit based on these methods include ISOGEN (manufactured by Nippon Gene Co., Ltd.), and TRIZOL reagent (manufactured by Invitrogen Corporation).

The resulting total RNA is used as a template to anneal an oligo dT primer to a poly A sequence of mRNA, and a reverse transcriptase such as RNaseH- Superscript II Reverse Transcriptase (manufactured by Invitrogen Corporation), and attached buffer and oligo dT primer are used to perform a reaction at 42°C for 1 hour and, then, this is heated at 99°C for 5 minutes to inactivate the reverse transcriptase. Then, mRNA-strand is nicked with RNaseH and, using the single-stranded cDNA as a template, double-stranded cDNA is synthesized with Escherichia coli DNA polymerase I. Ends of the resulting double-stranded cDNA are made blunt with T4 DNA polymerase. The blunt-ended double-stranded cDNA is inserted into pBluescript II vector or bacteriophage, for example, a vector such as λgt11, EMBL3 and the like, by T4 ligase to prepare a cDNA library. Examples of a commercially available kit based on these methods include cDNA Synthesis System Plus (manufactured by Amersham Biosciences Corporation) and TimeSaver cDNA Synthesis Kit (manufactured by Amersham Biosciences Corporation). From the thus prepared cDNA library, hybridization is performed, for example, using a DNA having a partial nucleotide sequence of the nucleotide sequence of any of SEQ ID NOs: 3, 4, 7 and 8 as a probe. Examples of the hybridization condition include the condition under which hybridization is performed under the stringent condition. Hybridization can be performed according to a conventional method described, for example, in Sambrook J., Frisch E.F., Maniatis T. , Molecular Cloning 2^{nd} edition, published by Cold Spring Harbor Laboratory Press. Examples of the "under the stringent condition" include the condition under which a hybrid is formed at 45°C in a solution containing 6xSSC (a solution containing 1.5 M NaCl and 0.15 M trisodium citrate is 10×SSC), and then washed at 50°C with 2xSSC (Molecular Biology, John Wiley & Sons, N.Y. (1989), 6.3.1-6.3.6), and the condition under which a hybrid is formed in a solution containing 50% formamide, 6xSSC, 5× Denhart solution, 0.5% (w/v) SDS and a heat-denatured salmon sperm DNA (100 µg/ml) by incubating at 42°C overnight using the aforementioned probe (10×10⁶ cpm/ml) labeled with [α-³²P]dCTP by a random priming method, and then washed in 2xSSC containing 0.1% (w/v) SDS at room temperature for 10 minutes and, further, washed in 0.2×SSC containing 0.1% (w/v) SDS at 55°C for 10 minutes twice. In addition, a salt concentration in the washing step may be selected, for example, from the condition of around 2xSSC at about 50°C (low stringency condition) to the condition of around 0.2xSSC and about 50°C (high stringency condition). A temperature at a washing step can be selected, for example, from room temperature (low stringency condition) to 65°C (high stringency condition). Alternatively, both of the salt concentration and temperature may be varied.

Then, a signal is detected with a X-ray film (e.g. Hyperfilm-MP; manufactured by Amersham Biosciences Corporation) or a bioimaging system (BAS-2000; manufactured by Fuji Photo Film Co. , Ltd.), and a recombinant containing a vector comprising a nucleotide sequence which binds with a probe can be obtained.

When a primer for a PCR method is designed, two may be selected from, for example, nucleotide sequences of SEQ ID NOs: 3, 4, 7 and 8, for example, so that the following conditions are satisfied.
1) A length of a primer is 15 bases to 40 bases, preferably 20 bases to 30 bases.
2) A ratio of guanine and cytosine in a primer is 40% to 60%, preferably 45% to 55%, more preferably 50% to 55%.
3) In a primer sequence, a distribution of adenine, thymine, guanine and cytosine is not partially biased. For example, a region where guanine and cytosine are repeated is not suitable.
4) A distance on a nucleotide sequence of a gene corresponding to a selected primer is preferably 100 bases to 3000 bases, further preferably 100 bases to 500 bases.
5) There is no complementary sequence in each primer itself, or between two primers.

Once a nucleotide sequence of a primer is selected, a primer may be chemically synthesized by a commercially available DNA synthesizer.

For example, there is a combination using a primer comprising the nucleotide sequence of SEQ ID NO: 11 as a sense primer, and a primer comprising the nucleotide sequence of SEQ ID NO: 12 as an antisense primer. As the condition for PCR, for example, primers are added to a reaction solution so that each of them becomes 200 nM, and PCR is performed by employing the above-synthesized single-stranded cDNA as a template and, for example, using LA Taq DNA polymerase (manufactured by TAKARA SHUZO Co., Ltd.) and a reaction buffer attached to the enzyme. As such the PCR, for example, heat denaturation is performed at 95°C for 3 minutes and, thereafter, around 35 cycles is performed, one cycle being 94°C for 30 seconds, 55°C for 30 seconds, and 72°C for 1 minute. Herein, in place of the cDNA synthesized as described above, a commercially available cDNA derived from various animals such as QUICK-Clone cDNA manufactured by Clontech Laboratories, Inc. may be used. An aliquot of the resulting reaction solution is analyzed by agarose gel electrophoresis, and an intended band is cloned into pGEM-T Easy vector (manufactured by Promega Corporation) by a TA cloning system, directly or after excision from the gel. A nucleotide sequence of the inserted DNA fragment can be determined and confirmed by the Die Terminator method.

The polynucleotide thus obtained having a nucleotide sequence encoding an amino acid sequence of the splicing mutant protein of the present invention (i.e. splicing mutant protein gene of the present invention) can be introduced into a vector which can be utilized in a host cell to be transformed. For example, a vector containing the splicing mutant protein gene of the present invention can be constructed by incorporating according to the conventional genetic engineering procedure into a vector which can be autonomously replicated in a host cell, which can be isolated and purified from a host cell, and which has a detectable marker. Examples of a vector containing the splicing mutant protein gene of the present invention may include plasmids such as pUC19 (manufactured by TAKARA SHUZO Co. , Ltd.) and pBluescript II (Stratagene Corporation), specifically when Escherichia coli is a host cell. When budding yeast is a host cell, plasmids such as pACT2 (manufactured by Clontech Laboratories, Inc.) and pYES2 (manufactured by Invitrogen Corporation) may be exemplified. In addition, when a mammal cell is a host cell, plasmids such as pRc/RSV and pRc/CMV (Invitrogen Corporation) may be exemplified.

A promoter operable in a host cell may be operably linked upstream of the splicing mutant protein gene of the present invention and incorporated into the aforementioned vector to construct a vector which can express the splicing mutant protein gene of the present invention. Herein, the "operably linked" means that the promoter and the splicing mutant protein gene of the present invention are linked so that the splicing mutant protein gene of the present invention is expressed under control of the promoter in a host cell into which the splicing mutant protein gene of the present invention is introduced. Examples of a promoter to be used include promoters exhibiting promoter activity in a host cell to be transformed and, for example, when a host cell is Escherichia coli, include promoters such as a promoter of lactose operon of Escherichia coli, tac promoter, T7 promoter and the like. When a host cell is an animal cell, examples include a Rouse sarcoma virus (RSV) promoter, Cytomegalovirus (CMV) promoter, a Simianvirus (SV40) promoter and the like. When a host cell is budding yeast, examples include alcohol dehydrogenase (ADH) 1 gene promoter, a galactose metabolism enzyme (GAL) 1 gene promoter and the like.

In addition, when a vector harboring in advance a promoter operable in a host cell is used, the splicing mutant protein gene of the present invention may be inserted downstream of the promoter so that the promoter harbored by the vector and the splicing mutant protein gene of the present invention are operably linked. For example, in the aforementioned plasmids, pRc/RSV, pRc/CMV and the like, a cloning site is provided downstream of a promoter operable in an animal cell and, when the splicing mutant protein gene of the present invention is inserted into the cloning site, and this is introduced into an animal cell, the splicing mutant protein gene of the present invention can be expressed. In addition, the aforementioned plasmid pACT2 for budding yeast has an ADH1 promoter and, when the splicing mutant protein gene of the present invention is inserted downstream of the ADH1 promoter of the plasmid or a derivative thereof, a vector of the present invention which can express the splicing mutant protein gene of the present invention at a large amount in budding yeast such as CG1945 strain (manufactured by Clontech Laboratories, Inc.) can be constructed.

By introducing the splicing mutant protein gene of the present invention or the vector of the present invention into a host cell, a transformant of the present invention can be obtained. As a method of introducing the splicing mutant protein gene of the present invention or the vector of the present invention into a host cell, a conventional introducing method can be applied depending on a host cell to be transformed. For example, when Escherichia coli which is a microorganism is a host cell, a conventional method can be used, such as a calcium chloride method, an electroporation method and the like described in Molecular Cloning 3^{rd} edition (Sambrook and Russell, Cold Spring Harbor Laboratory, 2001. In addition, when a mammal cell or an insect cell is a host cell, the gene or the vector can be introduced into the cell by a conventional gene transfection method such as a calcium phosphate method, an electroporation method and a lipofection method. Alternatively, the splicing mutant protein gene of the present invention may be expressed using yeast as a host cell. In this case, preferably, budding yeast (e.g. Saccharomyces cereviciae) is used, and yeast such as Pichia and the like may be also used. Examples of a method of transforming yeast include the method of Ito et al. (J. Bacteriol. 153; 163-168, 1983).

In order to incorporate the splicing mutant protein gene of the present invention into a virus such as Baculovirus and Vacciniavirus, a transfer vector can be used, which contains a nucleotide sequence homologous with a genome of a virus to be used. Examples of such the transfer vector include plasmids such as pVL1392, pVL1393 (manufactured by Invitrogen Corporation) and the like. When the splicing mutant protein gene of the present invention is inserted into the aforementioned transfer vector, and the transfer vector and a virus genome are introduced simultaneously into a host cell, homologous recombination occurs between the transfer vector and the virus genome, and a recombinant virus in which the splicing mutant protein gene of the present invention is incorporated into the genome can be obtained. As the virus genome, genomes of Baculovirus, Adenovirus and the like can be used. When a virus is used as a vector, as described above, a virus DNA can be introduced into a host cell by a conventional gene transfection method, or alternatively, a virus DNA can be also introduced into a host cell by directly infecting a host cell with a recombinant virus.

The splicing mutant protein of the present invention may be prepared as a natural protein by a procedure such as extraction and purification from a naturally occurring organism, or may be prepared as a recombinant protein by using a genetic engineering procedure. For example, a purified protein can be prepared by preparing a crude extract from a human cell or tissue, and using various columns. Herein, a cell is not particularly limited as far as it produces and expresses the splicing mutant protein of the present invention, but for example, a liver-derived cell, a kidney-derived cell and the like can be used. In addition, among the splicing mutant proteins of the present invention, a protein which is produced and expressed in an organism other than a human can be prepared from the organism.

In order to prepare the splicing mutant protein of the present invention, the splicing mutant protein gene of the present invention or the vector of the present invention is transformed into a suitable host cell as described above, and the transformant (i.e. transformant of the present invention) is cultured, thereby, a liver X receptor α splicing mutant protein may be produced. The produced liver X receptor α splicing mutant protein is recovered according to a conventional method. The recovered splicing mutant protein of the present invention is purified by a suitable method depending on the purpose. For example, when the transformant of the present invention is a microorganism, the transformant is cultured using various media appropriately containing a carbon source, a nitrogen source, organic salts, inorganic salts and the like, which are used in conventional culture in general microorganisms. Culturing is performed according to a conventional method in general microorganisms, and solid culture, liquid culture (test tube shaking culture, reciprocating shaking culture, Jar Fermenter culture, tank culture etc.) and the like are possible. A culture temperature can be appropriately changed in such a range that a microorganism is grown. For example, culture is generally performed at a culture temperature of about 15°C to about 40°C, in a culture medium at a pH of about 6 to about 8. A culture time is different depending on culture condition, and is usually about 1 hour to about 24 hours. When an inducible promoter is used, an induction time is desirably within one day, usually a few hours.

In addition, when the transformant is an animal cell such as a mammal, an insect and the like, the transformant can be cultured using a culture medium used in conventional culture in general cultured cells. In the case of an animal cell, for example, the cell may be cultured under the condition of 37°C and the presence of 5% CO₂ using a liquid medium (manufactured by such as Invitrogen Corporation) to which Fetal Bovine Serum (FBS) has been added to a final concentration of about 5% (v/v) to about 10% (v/v). When cells are grown to confluent, for example, an around 0.25% (v/v) trypsin/PBS solution is added to disperse into individual cells, this is diluted a few-fold and seeded on a new dish, and further cultured. In the case of an insect cell, similarly, for example, the cell may be cultured at a culture temperature of about 25°C to about 30°C using the Grace medium containing 10% (v/v) FBS or a serum-free medium such as SF-900 (manufactured by Invitrogen Corporation) and the like. In addition, when a recombinant virus vector such as Baculovirus is used, it is desirable to recover a cell within 72 hours after infection.

Recovering of the splicing mutant protein of the present invention produced by the transformant of the present invention may be performed by appropriate combination of conventional isolation and purification methods. For example, a fraction containing the intended splicing mutant protein of the present invention can be obtained by, after completion of culture, collecting cells of the transformant by centrifugation or the like, suspending the collected cells in a conventional buffer, for example, PBS containing an appropriate protease inhibitor, homogenizing the cells by ultrasonic treatment, a Dounce homogenizer or the like, centrifuging the homogenate at 20,000×g for a few tens of minutes to about 1 hour, and recovering the supernatant fraction. Further, the more purified intended splicing mutant protein of the present invention may be recovered from the supernatant fraction by subjecting to various chromatographies by the conventional protein purifying technique. In addition, in place of the splicing mutant protein of the present invention, the polypeptide of the present invention can be recovered by the similar method to that described above.

The thus prepared splicing mutant protein of the present invention can be used, for example, in a ligand-receptor binding assay for assessing binding ability and binding amount between a test substance and the splicing mutant protein of the present invention.

For example, a method for screening a substance which acts on a liver X receptor α splicing mutant protein comprises a step of contacting a test substance with a liver X receptor α splicing mutant protein, and a step of measuring a change in the liver X receptor α splicing mutant protein.

The liver X receptor α splicing mutant protein may be any liver X receptor α splicing mutant protein and, for example, the splicing mutant protein of the present invention can be exemplified as a particularly suitable protein.

In the aforementioned method, a concentration of the test substance to be contacted with the liver X receptor α splicing mutant protein may be usually about 0.1 µM to about 10 µM, preferably 1 µM to 10 µM. A time for contacting the liver X receptor α splicing mutant protein with the test substance is usually 18 hours or longer to around 60 hours, preferably 24 hours to around 40 hours.

The ligand-receptor binding assay using the splicing mutant protein of the present invention is a test method which enables analysis of binding specificity and binding strength, in addition to measurement of ability of a chemical substance to bind to the protein and quantitation of a binding amount. For example, in the state where a labeled ligand is bound to the splicing mutant protein of the present invention recovered from the transformant of the present invention as described above, when a test substance is allowed to coexist, the labeled ligand is released from the splicing mutant protein of the present invention due to competition between the test substance and the labeled ligand depending on affinity of both on the liver X receptor α splicing mutant protein. An amount of a labeled ligand bound to the splicing mutant protein of the present invention is reduced, and therefore, an amount of a label bound to the splicing mutant protein of the present invention is reduced. Thus, by monitoring an amount of a label of free-type labeled ligand or an amount of a label of binding-type labeled ligand, the ability of the test substance to bind to the splicing mutant protein of the present invention is indirectly indicated.

As a labeled ligand, for example, tritium-labeled oxycholesterol or the like may be used. A reaction system is roughly classified into three groups. One system is a group in which only a solvent is added to the state where a labeled ligand is bound to the splicing mutant protein of the present invention, this system corresponds to a system in which a concentration of a test substance is zero, and an amount of a label of binding-type labeled ligand obtained from this system indicates a total binding amount of the labeled ligand relative to the splicing mutant protein of the present invention. Another system is a system in which, for example, an unlabeled oxycholesterol is added to the state where the labeled ligand is bound to the splicing mutant protein of the present invention, to such a concentration (e.g. 10 µM) that the splicing mutant protein of the present invention is sufficiently saturated so as to have no capacity for binding to the labeled ligand. An amount of a label of binding-type labeled ligand obtained from this system is determined to be a non-specific binding amount of the labeled ligand relative to the splicing mutant protein of the present invention. Therefore, a specific binding amount of the labeled ligand relative to the splicing mutant protein of the present invention is a value obtained by subtracting the latter non-specific binding amount from the former total binding amount. A third system is a system in which a test substance is added to the state where the labeled ligand is bound to the splicing mutant protein of the present invention, for example, to a final concentration of 10 µM (this concentration is arbitrarily changed depending on the purpose). In the case where a test substance has ability to bind to the splicing mutant protein of the present invention, an amount of a label of a binding-type labeled ligand obtained from this system will be smaller than the above-obtained specific binding amount of the labeled ligand relative to the splicing mutant protein under the condition that concentration of the test substance is zero. By performing ligand-receptor binding assay in this way, ability of a test substance to bind to the splicing mutant protein of the present invention can be investigated and, when a test substance contains a plurality of substances, whether a substance exhibiting affinity for the splicing mutant protein of the present invention is present or not in those substances can also be investigated. Further, in order to assess ability of a test substance to bind to the splicing mutant protein of the present invention in more detail, for example, an assay is performed similarly under a different concentration of a test substance to be added in the third system, and an amount of a label of a binding-type labeled ligand is measured. Based on the measured value, amounts of binding-type and free-type ligands in respective assays are calculated, and binding affinity, binding specificity, binding capacity and the like between the test substance and the splicing mutant protein of the present invention can be assessed.

In addition, whether a liver X receptor α splicing mutant protein activates transcription of a gene under the control of a transcription regulatory region containing a liver X receptor α-binding sequence or not can be assessed, for example, by using a reporter gene linked downstream of a transcription regulatory region containing a liver X receptor α-binding sequence by a test method such as reporter assay described later. By forming a heterodimer with a retinoid X receptor (RXR) that is one of nuclear receptors, and binding to the transcription regulatory region containing a liver X receptor α-binding sequence, a liver X receptor α regulates transcription of a gene under the control of the transcription regulatory region. Examples of the liver X receptor α-binding sequence include a particular sequence (cis sequence) of a transcription regulatory region containing a core motif having a structure in which two "AGGTCA"s (or a motif consisting of related six nucleotides) are aligned in the same direction. A spacer between core motifs is different depending on a combination of heterodimers, and a spacer for a heterodimer in the case of a liver X receptor and a retinoid receptor is 4 bp (DR4) (e.g. see Lehman et al., J.Biol.Chem., 272; 3137-3140, 1997).

The liver X receptor α splicing mutant proteins of the present invention, genes thereof, or a portion thereof are extremely useful for elucidating relationship between the liver X receptor α splicing mutant protein and disease symptoms with which the function of the protein is directly or indirectly involved, and developing a method and an agent useful for preventing, diagnosing or treating the disease symptom. By the aforementioned screening method, a substance which acts on the liver X receptor α splicing mutant protein, and an antagonist to the substance can be searched, and these substances can be provided as a candidate for a medicament.

An agent containing, as an active ingredient, a substance selected by the screening method of the present invention or an antagonist to the substance can be administered to a mammal such as a human and the like orally or parenterally at its effective amount. For example, when orally administered, the agent can be used in a conventional form such as tablets, capsules, syrups, suspensions and the like. In addition, when administered parenterally, the agent can be used in a conventional liquid form such as solutions, emulsions, suspensions and the like. Examples of a method of parenterally administering the agent (the adipose accumulation regulator) in the aforementioned form include a method of injecting it, and a method of administering to a rectum in a form of suppositories.

The aforementioned suitable dosage form can be prepared by blending a substance selected by the screening method of the present invention or an antagonist to the substance into conventional acceptable carriers, excipients, binders, stabilizers, diluents or the like. In addition, when used in an injection form, acceptable buffers, solubilizers, isotonics and the like may be added.

A dose is different depending on an age, a sex, a weight, and an extent of a disease of a mammal to be administered, a kind and an administration form of the agent and the like and, usually, in the case of oral administration, an amount of an active ingredient of about 1 mg to about 2 g, preferably about 5 mg to about 1 g may be administered a day per adult. In the case of injection, an amount of an active ingredient of about 0.1 mg to about 500 mg may be administered per adult. In addition, the aforementioned dose per day can be administered once or by dividing into a few times.

Relationship with disease symptom with which the splicing mutant protein of the present invention is directly or indirectly involved can be elucidated by first specifying a ligand and, then, specifying a gene cluster, transcription of which is regulated by the ligand and the splicing mutant protein of the present invention. Specification of a ligand can be performed by detecting a change in an expression level of a gene cluster, transcription of which is regulated by the splicing mutant protein of the present invention or the polypeptide of the present invention, by contacting a test substance with the splicing mutant protein of the present invention or the polypeptide of the present invention.

Alternatively, a method for screening a substance which acts on a liver X receptor α splicing mutant protein may be a method comprising a step of contacting a test substance with a cell producing a liver X receptor α splicing mutant protein, a step of measuring an amount of production of other protein which undergoes expression regulation by the liver X receptor α spicing mutant protein, and a step of assessing acting property of the test substance on the liver X receptor α splicing mutant protein based on the amount of production.

Herein, the cell producing a liver X receptor α splicing mutant protein may be any cell producing a liver X receptor α splicing mutant protein, and particularly suitable examples include a cell producing the splicing mutant protein of the present invention. A concentration of a test substance to be contacted with a cell producing a liver X receptor α splicing mutant protein may be usually about 0.1 µM to about 10 µM, preferably 1 µM to 10 µM. A time for contacting a test substance with a cell producing a liver X receptor α splicing mutant protein is usually 18 hours or longer to around 60 hours, preferably 24 hours to around 40 hours.

In addition, other examples of a method for screening a substance which acts on a liver X receptor α splicing mutant protein may include a method comprising a first step of contacting a cell producing a liver X receptor α splicing mutant protein with a ligand for the liver X receptor α splicing mutant protein, and measuring an amount of production of other protein which undergoes expression regulation by the liver X receptor α splicing mutant protein, a second step of contacting a cell producing the splicing mutant protein of the present invention with both of the ligand for the liver X receptor α splicing mutant protein and a test substance, and measuring an amount of production of other protein which undergoes expression regulation by the liver X receptor α splicing mutant protein, and a step of assessing antagonizing property of the test substance against the ligand based on a difference in the amounts of production of the aforementioned other proteins measured in the first step and the second step. Herein, the liver X receptor α splicing mutant protein may be any liver X receptor α splicing mutant protein, and particularly suitable examples include the splicing mutant protein of the present invention. A concentration of a ligand or a test substance to be contacted with a cell producing a liver X receptor α splicing mutant protein may be usually about 0.1 µM to about 10 µM, preferably 1 µM to 10 µM. A time for contacting the cell producing a liver X receptor α splicing mutant protein with the ligand or the test substance is usually 18 hours or longer to around 60 hours, preferably 24 hours to around 40 hours.

Further, the generally performed binding assay can be performed using the splicing mutant protein of the present invention or a fragment thereof. Other example includes a method of constructing an expression system of the splicing mutant protein of the present invention and a target gene to which the liver X receptor α splicing mutant protein is bound, and detecting increase due to ligand addition in a protein which is a target gene product. For example, by using a reporter gene which expresses a reporter protein such as lucif erase, chloramphenicol acetyltransf erase, β-galactosidase and the like under control of a promoter of a target gene, the presence or the absence of expression of a target gene in a host cell can be easily detected. Further, in place of a full length of the splicing mutant protein of the present invention, a chimeric gene of a ligand binding region of said protein and a DNA binding protein is expressed, and a plasmid in which a minimum active promoter and a gene encoding the aforementioned reporter protein are ligated downstream of a nucleotide sequence to which the DNA binding protein is bound, can be used as a reporter gene. As the DNA binding protein, for example, GAL4, LexA and the like can be used. An expression system of the splicing mutant protein of the present invention and a target gene to which the liver X receptor α splicing mutant protein is bound, and a plasmid for expressing the chimeric gene of a ligand binding region of the protein and a DNA binding protein can be prepared by the conventional genetic engineering technique.

Further, alternatively, by transcribing a labeled DNA from mRNA expressed in a cell, and hybridizing the resulting labeled DNA with a DNA library chip, a gene expression of which has been regulated by the splicing mutant protein of the present invention may be specified.

Relationship with disease symptom with which the splicing mutant protein of the present invention is directly or indirectly involved can be elucidated by specifying a gene to be activated, by comparing an expression amount of a target gene when an antagonist or agonist is contacted with a cell expressing the splicing mutant protein of the present invention, and an expression amount of the target gene in the cell expressing the splicing mutant protein of the present invention with which the test substance is not contacted.

Examples of a method for detecting the presence or the absence of the splicing mutant protein of the present invention include a method comprising a first step of synthesizing complementary DNA (cDNA) from messenger RNA (mRNA) in a biological sample, a second step of amplifying a portion of the complementary DNA (cDNA) corresponding to the splicing mutant protein gene of the present invention or the polypeptide gene of the present invention, and a third step of detecting the amplified portion.

Specific examples of the present splicing mutant protein gene or the present polypeptide gene include a polynucleotide comprising a nucleotide sequence of SEQ ID NO: 3, 4, 7 or 8.

In addition, in the second step, a preferable example includes a step of amplification by a PCR method using, as primers, one or a plurality selected from polynucleotides comprising any of nucleotide sequences of SEQ ID NO: 9, SEQ ID NO: 10, SEQ ID NO: 11, SEQ ID NO: 12, SEQ ID NO: 13 and SEQ ID NO: 14.

In addition, regarding an experimental animal such as a gene-defective animal in which ability to express a polynucleotide comprising a nucleotide sequence encoding an amino acid sequence of the splicing mutant protein of the present invention is deficient, and utilization thereof, a model animal is produced by destructing (inactivating) a gene comprising a nucleotide sequence encoding an amino acid sequence of an endogenous splicing mutant derived from an experimental animal, said gene having the function of a gene comprising a nucleotide sequence encoding an amino acid sequence of the splicing mutant protein of the present invention, and physical, biological, pathological and genetic characteristics of this model animal are analyzed, thereby, it becomes possible to elucidate relationship between the function of the splicing mutant protein of the present invention and diseases.

In addition, by introducing the human-derived gene of the present invention into a model animal in which a gene comprising a nucleotide sequence encoding an amino acid sequence of the aforementioned endogenous liver X receptor α splicing mutant protein has been destructed (inactivated), a model animal having only the human-derived gene of the present invention is produced, and a agent (compound etc.) targeting the introduced human-derived gene is administered, thereby, it is possible to assess therapeutic effect of the agent.

The present invention further provides use of a DNA probe specific for a nucleotide sequence of the polypeptide gene of the present invention for gene therapy, and use of a RNA probe specific for a nucleotide sequence of the polypeptide gene of the present invention. A probe for diagnosing a disease involved in inhibition of normal cholesterol metabolism by a normal liver X receptor α, consisting a whole or a part of a gene (DNA or RNA) comprising a nucleotide sequence encoding an amino acid sequence of such the splicing mutant protein of the present invention (or a nucleotide sequence complementary to the nucleotide sequence), and an agent containing the probe for diagnosing a disease accompanied with the disorder are also provided. For example, by performing In situ hybridization of the splicing mutant protein gene on a pathological section, the presence or the absence and a progression degree of a disease accompanied with the disorder can be detected.

Herein, a diagnostic probe is not particularly limited as far as it is a whole or a part of an antisense strand of the splicing mutant protein gene (DNA, RNA, cDNA) of the present invention, and has a length to such an extent that it is constituted as a probe (at least 20 bases or longer). In order to use the probe as an active ingredient of a diagnostic, it is preferable to dissolve the probe in a suitable buffer or sterilized water in which it is not degraded. In addition, examples of a method of In situ hybridization include a procedure described, for example, in J. Neurobiol. 29, 1-17 (1996). Alternatively, it is also possibly to utilize an In situ PCR method.

In the aforementioned diagnosis, not only a probe, but also an antibody (see below) specifically recognizing the splicing mutant protein of the present invention can be used and, in this case, the presence or the absence of , and a progression extent of diseases involved in inhibition of normal cholesterol metabolism due to a normal liver X receptor α can be detected by an immunostaining method (see Dev. Biol. 170, 207-222 (1995); J. Neurobiol. 29, 1-17 (1996)). An antibody to be used can be prepared, for example, by the conventional method described in Antibodies; A Laboratory Manual edited by Lane, H.D. et al., published by Cold Spring Harbor Laboratory Press, New York 1989.

The splicing mutant protein gene of the present invention or the polypeptide gene of the present invention itself is useful as an antisense medicament for controlling the function of the splicing mutant protein of the present invention at a gene level, in use in gene therapy. For example, a nucleotide sequence of 20 bases or more, preferably a nucleotide sequence of around 30 bases contained in SEQ ID NO: 7 or 8 can be used.

The aforementioned antisense medicament includes not only a DNA but also an RNA. In addition, a nucleotide sequence may be a sense sequence or an antisense sequence (i.e. a nucleotide sequence complementary to the sense sequence).

Herein, the "polypeptide of the present invention" or the "polypeptide gene of the present invention" is, as described above, a portion of the splicing mutant protein of the present invention (partial polypeptide) or a portion of the splicing mutant protein gene of the present invention (partial polynucleotide) corresponding thereto, and means a polypeptide comprising a region which is not present in a normal live X receptor α, or a polynucleotide corresponding thereto. It is preferable that at least 6 consecutive amino acids or at least 18 consecutive bases are contained.

Representative examples include a polypeptide comprising a region which is not present in a normal liver X receptor α among an amino acid sequence of an isoform of a liver X receptor α in which intron 5 of a liver X receptor α gene is contained as an exon (splicing mutant protein 5A of the present invention), or a polynucleotide corresponding thereto, and a polypeptide comprising a region which is not present in a normal liver X receptor α among an amino acid sequence of an isoform of a liver X receptor α in which a portion in intron 6 of a liver X receptor α gene is contained as an exon (splicing mutant protein 6A of the present invention), or a polynucleotide corresponding thereto.

Further, specific examples include a polypeptide comprising the amino acid sequence of SEQ ID NO: 5 or a polynucleotide comprising a nucleotide sequence (e.g. nucleotide sequence of SEQ ID NO: 7) corresponding thereto, and a polypeptide comprising the amino acid sequence of SEQ ID NO: 6 or a polynucleotide comprising a nucleotide sequence (e.g. nucleotide sequence of SEQ ID NO: 8) corresponding thereto.

In addition, a recombinant vector containing a polynucleotide comprising a nucleotide sequence encoding an amino acid sequence of the aforementioned polypeptide or the aforementioned polynucleotide may be prepared according to the aforementioned method of preparing the vector of the present invention. Further, by introducing the thus prepared recombinant vector into a host cell according to the aforementioned method of preparing the transformant of the present invention, a transformant can be also prepared.

Among nucleotide sequences encoding amino acid sequences of the splicing mutant proteins of the present invention, the nucleotide sequence of SEQ ID NO: 7 or 8 can be utilized as a tool for searching a further liver X receptor α splicing mutant protein other than the splicing mutant protein of the present invention as a probe or a primer. It is desirable that the nucleotide sequence is a nucleotide sequence having a length containing at least 15 consecutive bases. A probe can be labeled with a radioactive isotope, digoxigenin, biotin, a detectable enzyme or the like by the conventional method. For example, in the case where, radiolabeled phosphorus is used, when a cDNA fragment is used, it is convenient to label it with a random priming label and, when a synthetic primer is used, it is convenient to label a 5'-end with a phosphorylation enzyme. The thus labeled probe is hybridized with a cDNA library to perform cloning. Hybridization can be performed by the conventional method and under the conventional condition. Examples include washing in 1×SSC, 0.5% (w/v) SDS at 65°C. The cDNA library may be derived from an animal including a mammal , and a library derived from a human tissue or cell is particularly desirable.

An antibody specifically recognizing the splicing mutant protein of the present invention or a part of the antibody can be prepared.

An antibody specifically recognizing the splicing mutant protein of the present invention comprising the amino acid sequence of SEQ ID NO: 1, 2, 5 or 6 can be obtained, for example, by immunizing an animal usually used for producing an antibody such as rabbit, guinea pig, rat, mouse, goat and sheep, using the splicing mutant protein of the present invention obtained by the aforementioned conventional genetic engineering method or a portion (polypeptide) thereof, or a peptide fragment comprising the amino acid sequence of SEQ ID NO: 5 or 6. In addition, a monoclonal antibody can be obtained by fusing a spleen cell of an immunized mammal and a myeloma cell.

Examples of a method of detecting the presence or the absence of the splicing mutant protein of the present invention utilizing these antibodies include a method comprising a step of detecting the aforementioned liver X receptor α splicing mutant protein based on an antigen-antibody reaction using an antibody which can specifically detect the splicing mutant protein of the present invention or the polypeptide of the present invention. Further specifically, examples include a method for detecting the presence of the absence of the splicing mutant protein of the present invention comprising a first step of binding to a microtiter well a primary antibody which can specifically detect the splicing mutant protein of the present invention or the polypeptide of the present invention, a second step of binding to the primary antibody on the microtiter well the splicing mutant protein of the present invention in a biological sample, a third step of removing all biological samples in the non-bound state (i.e. free) in the microtiter well after the second step, a forth step of binding to a liver X receptor α splicing mutant protein bound to the primary antibody a labeled secondary antibody which can bind to a different epitope from that of the primary antibody, a fifth step of removing all secondary antibodies in the non-bound state (i.g. free) in the microtiter well after the forth step, and a sixth step of detecting the presence or the absence of the secondary antibody using a label of the secondary antibody as an index after the fifth step.

### Examples

The present invention will be explained in more detail by way of the following Examples, but the present invention is not limited to these Examples.

### Example 1 (Cloning of liver X receptor α splicing mutant protein gene)

Using a cDNA derived from each human tissue (Multiple Tissue cDNA (MTC) Panels, manufactured by Clontech Laboratories, Inc.) as a template, a region corresponding to exon 4 to exon 8 of a human liver X receptor α gene was amplified by a PCR method using a programmable thermal cycler. As primers, a primer comprising a nucleotide sequence of a sense strand of exon 4 (SEQ ID NO: 9, LXRαF), and a primer comprising a nucleotide sequence of an antisense strand of exon 8 (SEQ ID NO: 10, LXRαR) were designed and chemically synthesized based on a nucleotide sequence of a human liver X receptor α gene (Willy et al., Genes Dev.9: 1033-1045, 1995; GenBank Accession number, NM_005693). The reaction condition of the PCR method was as follows. A 50 µl of a reaction solution (containing MgCl₂, and a deoxyribonucleotide mixed solution) containing 1.25 unit of Ex Taq DNA Polymerase (manufactured by TAKARA SHUZO Co., Ltd.), 200 nmol of the sense primer LXRαF (SEQ ID NO: 9), 200 nmol of the antisense primer LXRαR (SEQ ID NO: 10) and single-stranded cDNA which is a template was prepared. This reaction solution was heat-denatured at 95°C for 3 minutes, and was subjected to a reaction of 35 cycles, one cycle being 94°C for 30 seconds, 55°C for 30 seconds and 74°C for 60 seconds. The resulting PCR product was electrophoresed on a 1% agarose gel, amplification of a DNA fragment was confirmed, and 1 µl of the PCR product was ligated to pGEM-T Easy vector (manufactured by Promega Corporation) using a TA cloning system. The ligation was performed according to the method which is recommended for a kit used. Competent cells of Escherichia coli JM109 strain (manufactured by TAKARA SHUZO Co., Ltd.) were transformed with the resulting ligation reaction solution by the method recommended by the manufacturer. The resulting bacterial cells were spread on a LB agar medium containing ampicillin on which X-gal and isopropyl β-D-glactopyranoside (IPTG) hadbeen coated, and this was cultured at 37°C overnight. After culturing, the ampicillin-resistant white colony formed on the agar medium was inoculated in 3 ml of a LB liquid medium containing ampicillin, and this was cultured to grow at 37°C overnight. A plasmid DNA was extracted and isolated from the grown Escherichia coli according to a conventional method. The isolated plasmid DNA was cut with a restriction enzyme Eco RI, and this was subjected to 1% agarose gel electrophoresis. After electrophoresis, a size of the inserted DNA fragment was confirmed under ultraviolet-ray irradiation. Further, a nucleotide sequence of the inserted DNA fragment was determined. Like this, a liver X receptor α splicing mutant protein gene was cloned.

### Example 2 (Identification of band of liver X receptor α: presence of selective splicing transcript)

Using primers (LXRαF and LXRαR) specific for a liver X receptor α, comprising either of nucleotide sequences of SEQ ID NOs: 9 and 10, and using a cDNA derived from a human normal tissue or a human cancer tissue as a template, a DNA fragment was amplified. Results are shown in Fig. 2. A band of 506 base pairs indicated by an arrow corresponded to an amplified band derived from the known normal liver X receptor α gene. It was revealed that, besides this, two kinds of bands which do not disappear even when reaction condition of PCR was changed, that is, a band of a lower-molecular weight and a band of a higher molecular weight are present (see Fig. 2). These bands suggest a possibility that selective splicing transcripts of a liver X receptor α are present.

### Example 3 (Cloning of liver X receptor α splicing mutant protein gene)

The PCR products associated with liver X receptor α were cloned, and their nucleotide sequences were determined. As PCR primers for amplification and cloning, the sense primer (LXRαF, SEQ ID NO: 9) and the antisense primer (LXRαR, SEQ ID NO: 10) used in Example 1 were used. PCR was performed using cDNAs derived from a human normal tissue and a human cancer tissue as a template, the resulting PCR product was directly ligated to pGEM-T Easy vector (manufactured by Promega Corporation) by a TA cloning method. As in Example 1, after a size of the inserted DNA fragment was confirmed, a nucleotide sequence of the inserted DNA fragment was further determined. As a result, it was revealed that in addition to DNA encoding the liver X receptor α splicing mutant protein D5 in which an amino acid sequence encoded by exon 5 is deleted from a liver X receptor α derived from a human normal tissue, newly, DNAs encoding two novel liver X receptor α splicing mutant proteins (a liver X receptor α splicing mutant protein in which an amino acid sequence encoded by intron 5 is inserted, and a liver X receptor α splicing mutant protein in which an amino acid sequence encoded by a portion of intron 6 is inserted) are present.

### Example 4 (Identification of a liver X receptor α splicing mutant protein in which an amino acid sequence encoded by intron 5 is inserted)

In order to isolate a whole translated region encoding a liver X receptor α splicing mutant protein in which an amino acid sequence encoded by intron 5 is inserted, primers which can amplify a whole translated region were newly designed. Using hLXRα(ORF)-F/H (SEQ ID NO: 11) as a sense primer and hLXRα(ORF)-R/B (SEQ ID NO: 12) as an antisense primer, and using a human liver-derived cDNA (manufactured by Clontech Laboratories, Inc.) as a template, a DNA fragment was amplified by a PCR method. The reaction condition of the PCR method was as follows. 50 µl of a reaction solution (containing MgCl₂, a deoxyribonucleotide mixed solution) containing 1.25 unit of Ex Taq DNA Polymerase (manufactured by TAKARA SHUZO Co. , Ltd.), 200 nmol of the sense primer hLXRα(ORF)-F/H (SEQ ID NO: 11), 200 nmol of the antisense primer hLXRα(ORF)-R/B (SEQ ID NO: 12) and a single-stranded cDNA which is a template (liver-derived cDNA manufactured by Clontech Laboratories, Inc.) was prepared. This reaction solution was heat-denatured at 96°C for 3 minutes, and was subjected to a reaction of 35 cycles, one cycle being 96°C for 30 seconds, 55°C for 30 seconds and 72°C for 90 seconds. An aliquot of the resulting PCR product was electrophoresed on a 1% agarose gel containing ethidium bromide, amplification of a DNA fragment was confirmed, and 1 µl of the PCR product was ligated to pGEM-T Easy vector (manufactured by Promega Corporation) using a TA cloning system. The ligation was performed according to the method recommended by a kit used. Competent cells of Escherichia coli JM109 strain (manufactured by TAKARA SHUZO Co., Ltd.) were transformed by the resulting ligation reaction solution according to the method recommended by the manufacturer. The resulting bacterial cells were spread on a LB agar medium containing ampicillin on which X-gal and isopropyl β-D-galactopyranoside (IPTG) had been coated, and this was cultured at 37°C overnight. After culturing, a transformant which had formed an ampicillin-resistant white colony on the agar medium was used to perform colony PCR, thereby, a clone containing a whole translated region encoding a liver X receptor α splicing mutant protein in which intron 5 had been inserted was detected. The reaction condition of the colony PCR method was as follows. As a sense primer, LXRαF (SEQ ID NO: 9) was used and, as an antisense primer, a specific primer LXRα5A-R(SEQ ID NO: 13) which had been designed based on a nucleotide sequence in intron 5 was used. A reaction solution containing these two kinds of primers, Ex Taq DNA Polymerase (manufactured by TAKARA SHUZO Co. , Ltd.), and 200 nM deoxyribonucleotide mixed solution was prepared. A small amount of the aforementioned transformant exhibiting white which had been taken from the plate was suspended therein, this reaction solution was heat-denatured at 95°C for 5 minutes, and was subjected to a reaction of 25 cycles, one cycle being 94°C for 30 seconds, 55°C for 30 seconds, and 74°C for 30 seconds. The resulting PCR product was electrophoresed on an agarose gel containing ethidium bromide, amplification of a DNA fragment was confirmed, and a plasmid was extracted from the transformant, for which amplification of the DNA fragment had been confirmed. By confirming a nucleotide sequence of a DNA fragment inserted into the plasmid, a clone containing a whole translated region encoding a liver X receptor α splicing mutant protein in which intron 5 had been inserted was obtained. The liver X receptor α splicing mutant protein was such that an amino acid sequence encoded by intron 5 is inserted into a normal liver X receptor α, and was named as liver X receptor α splicing mutant protein 5A. Fig. 1 shows comparison of a translated region encoding a human normal liver X receptor α and a translated region encoding the liver X receptor α splicing mutant protein 5A. As compared with a translated region encoding a human normal liver X receptor α, a different point of the translated region encoding the liver X receptor α splicing mutant protein 5A is only a point that intron 5 is contained in a translated region, and no difference was recognized in other nucleotide sequence. Since even when intron 5 is inserted between exons 5 and 6 as an additional translated region, the number of inserted bases (SEQ ID NO: 7) is 192 (encoding 64 amino acids represented by SEQ ID NO: 5), that is, a multiple of 3, a reading frame after exon 6 was entirely the same as that of a normal type. An amino acid sequence derived from the translated region encoding a liver X receptor α splicing mutant protein 5A becomes a protein of 511 amino acids (1533 bases). Among an amino acid sequence of the liver X receptor α splicing mutant protein, homology of an amino acid sequence (SEQ ID NO: 5) encoded by intron 5 was searched using genome database and protein database, and it was found out that an amino acid sequence having more than 30% homology is not present in the databases. From this, it was confirmed that the amino acid sequence (SEQ ID NO: 5) encoded by intron 5, of the liver X receptor α splicing mutant protein is novel.

### Example 5 (Identification of a translated region encoding a liver X receptor α splicing mutant protein, containing a novel nucleotide sequence (109 bases) added downstream of exon 6)

In order to isolate a whole translated region encoding a liver X receptor α splicing mutant protein, in said region a portion of intron 6 being inserted, as in Example 4, a DNA fragment was amplified by a PCR method using hLXRα(ORF)-F/H (SEQ ID NO: 11) as a sense primer and hLXRα(ORF)-R/B (SEQ ID NO: 12) as an antisense primer, and using a human liver-derived cDNA (manufactured by Clontech Laboratories, Inc.) as a template.

The reaction condition of the PCR method was as follows. 50 µl of a reaction solution (containing MgCl₂, and a deoxyribonucleotide mixed solution) containing LA Taq DNA Polymerase (manufactured by TAKARA SHUZO Co., Ltd.), 200 nmol of the sense primer, 200 nmol of the antisense primer and a single-stranded cDNA which is a template (liver-derived cDNA manufactured by Clontech Laboratories, Inc.) was prepared. This reaction solution was heat-denatured at 96°C for 3 minutes, and was subjected to a reaction of 35 cycles, one cycle being 96°C for 30 second, 55°C for 30 seconds, and 72°C for 90 seconds. An aliquot of the resulting PCR product was electrophoresed on an agar gel containing ethidium bromide, amplification of a DNA fragment was confirmed, and the PCR product was ligated to pGEM-T-Easy vector (manufactured by Promega Corporation) using a TA cloning system. The ligation was performed according to the method recommended by a kit used. Competent cells of Escherichia coli JM109 strain (manufactured by TAKARA SHUZO Co., Ltd.) were transformed by the resulting ligation reaction solution according to the method recommended by the manufacturer. The resulting bacterial cells were spread on a LB agar medium containing ampicillin on which X-gal and isopropyl β-D-galatcopyranoside (IPTG) had been coated, and this was cultured at 37°C overnight. After culturing, by performing colony PCR using the transformant which had formed an ampicillin-resistant white colony on the agar medium, a clone containing a whole translated region encoding a liver X receptor α splicing mutant protein in which intron 6 had been inserted was detected. The reaction condition of the colony PCR method was as follows. As a sense primer, LXRαF (SEQ ID NO: 9) was used and, as an antisense primer, a specific primer LXRα6A-R (SEQ ID NO: 14) which had been designed based on a nucleotide sequence in intron 6 was used. 30 µl of a reaction solution containing these two kinds of primers, Ex Taq DNA polymerase (manufactured by (TAKARA SHUZO Co., Ltd.), and 200 nM deoxyribonucleotide mixed solution was prepared. A small amount of the transformant exhibiting white which had been taken from the plate was suspended therein, this reaction solution was heat-denatured at 95°C for 5 minutes, and was subjected to a ration of 25 cycles, one cycle being 94°C for 30 seconds, 55°C for 30 seconds, and 74°C for 30 seconds. The resulting PCR product was electrophoresed on an agarose gel containing ethidium bromide, amplification of a DNA fragment was confirmed, and a plasmid was extracted from the transformant, for which amplification of the DNA fragment had been confirmed. By confirming a nucleotide sequence of a DNA fragment which had been inserted into the plasmid, a clone containing a whole translated region containing a liver X receptor α splicing mutant protein in which a portion of intron 6 had been inserted was obtained. The liver X receptor α splicing mutant protein was named as liver X receptor α splicing mutant protein 6A for indicating that a nucleotide sequence of a portion of intron 6 (i.e. nucleotide sequence called exon 6A) is encoded by a translated region inserted downstream of exon 6. Fig. 1 shows comparison between a translated region encoding a human normal liver X receptor α and a translated region encoding the liver X receptor α splicing mutant protein 6A. As compared with a translated region encoding a human normal liver X receptor α, a different point of the translated region encoding the liver X receptor α splicing mutant protein 6A is only a point that a portion of intron 6 is contained in the translated region, and no difference was recognized in other nucleotide sequence. Among a nucleotide sequence of intron 6, when a region of 109 base pairs (SEQ ID NO: 8) is inserted between exons 6 and 7 as an additional translated region, since a new stop codon appears in midstream, consequently, new 26 amino acids (SEQ ID NO: 6) are ligated downstream of an amino acid sequence encoded by exon 6. That is, a new polypeptide of 356 amino acids (SEQ ID NO: 2) is produced. Among an amino acid sequence of the liver X receptor α splicing mutant protein, homology of an amino acid sequence encoded by a portion of intron 6 (SEQ ID NO: 6) was searched using genome database and protein database, and it was found out that an amino acid sequence having more than 30% homology is not present in the databases. From this, it was confirmed that the amino acid sequence (EQ ID NO: 6) encoded by a portion of intron 6 of the liver X receptor α splicing mutant protein is novel.

### Example 6 (Presence of transcripts of three kinds of liver X receptor α splicing mutant protein genes in a human tissue)

Whether three liver X receptor α splicing mutant proteins found out by the present invention are present in RNA derived from human normal tissues and human cancer tissues or not is confirmed. Using primers comprising either of nucleotide sequences of SEQ ID NOs: 9, 10, 13 and 14 which specifically amplify respective liver X receptor α splicing mutant proteins, and using cDNAs derived from a human normal tissue and a human cancer tissue as a template, PCR was performed. When LXRαF (SEQ ID NO: 9) and LXRαR (SEQ ID NO: 10) are used as primers in the PCR, amplification of a DNA fragment derived from a normal liver X receptor α results in amplification of a DNA fragment of 506 base pairs, and amplification of a DNA fragment derived from the liver X receptor αD5 results in amplification of a DNA fragment of 416 base pairs. On the other hand, when PCR is performed as described above using a primer comprising the nucleotide sequence of SEQ ID NO: 9 and a primer comprising the nucleotide sequence of SEQ ID NO: 13 for specifically amplifying the liver X receptor αsplicing mutant protein 5A, amplification of a DNA fragment derived from the liver X receptor α splicing mutant protein 5A results in amplification of a DNA fragment of 432 base pairs and, when PCR is performed as described above using a primer comprising the nucleotide sequence of SEQ ID NO: 9 and a primer comprising the nucleotide sequence of SEQ ID NO: 14 for specifically amplifying the liver X receptor α splicing mutant protein 6A, amplification of a DNA fragment derived from a liver X receptor α splicing mutant protein 6A results in amplification of a DNA fragment of 428 base pairs. As a control, in order to check DNA contamination in regents, PCR was performed in the absence of a template DNA. Results were shown in Fig. 2, Fig. 3 and Fig. 4.

As apparent from these figures,
1) The normal liver X receptor α was expressed in any of human normal tissues and human cancer tissues.
2) The liver X receptor α splicing mutant protein 5A was expressed in some of human cancer tissues.
3) The liver X receptor α splicing mutant protein 6A was highly expressed in human circulatory tissues.
4) The liver X receptor α splicing mutant protein D5 was expressed in human cancer tissues at a high level.

The control was negative at all times. These results show that each of liver X receptor α splicing mutant proteins is tissue-specifically expressed, and it was found out that the protein undergoes specific expression regulation.

### Example 7 (Construction of a liver X receptor α splicing mutant protein-expressing vector)

A plasmid into which each of a normal liver X receptor α and three kinds of liver X receptor α splicing mutant proteins had been cloned was cut with restriction enzymes Hind III and BamH I according to the method recommended by a manufacturer. A solution treated with the enzymes was subjected to agarose gel electrophoresis, and a DNA fragment corresponding to an insert DNA was recovered from the gel. The recovered DNA fragment was cloned into pFLAG-CMV2 (Sigma) which had been cut with Hind III and BamH I in advance. The pFLAG-CMV2 (about 300 ng) which had been cut with Hind III and BamH I and the insert DNA (about 500 ng) were mixed, T4 ligase was added thereto, and the mixture was ligated at 16°C for 30 minutes. The ligation was performed according to the method recommended by a kit used. Competent cells of Escherichia coli JM109 strain (manufactured by TAKARA SHUZO Co., Ltd.) were transformed by the resulting ligation reaction solution according to the method recommended by the manufacturer. The resulting bacterial cells were spread on a LB agar medium containing ampicillin on which X-gal and isopropyl β-D-galactopyranoside (IPTG) had been coated, and this was cultured at 37°C overnight. After culturing, a plasmid DNA was prepared from the ampicillin-resistant white colony formed on the agar medium. Further, a nucleotide sequence of the inserted DNA fragment was determined. The resulting nucleotide sequence was compared with the aforementioned nucleotide sequence obtained by direct sequencing, a plasmid in which a nucleotide sequence of a translated region had been confirmed to be identical was selected, and respective plasmids were named as pCMV2-hLXRαWT, pCMV2-hLXRα5A, pCMV2-hLXRα6A, and pCMV2-hLXRαD5.

### Example 8 (Preparation of a plasmid containing a luciferase reporter gene having a liver X receptor α-binding sequence)

Two oligonucleotides comprising a nucleotide sequence derived from a nucleotide sequence adjacent to TATA box and a leader sequence of mouse metallothionein I gene, that is, an oligonucleotide comprising the nucleotide sequence of SEQ ID NO: 15 and an oligonucleotide comprising the nucleotide sequence of SEQ ID NO: 16 were annealed to form a double-stranded DNA, and T4 polynucleotide kinase was acted on this to phosphorylate both ends thereof (hereinafter, the DNA is referred to as TATA DNA). Separately, a plasmid pGL3-Basic vector (manufactured by Promega Corporation) containing a firefly luciferase gene was digested with restriction enzymes Bgl II and Hind III, bacterial alkaline phosphatase (BAP) was added thereto, followed by incubation at 65°C for 1 hour. Then, the incubated solution was subjected to electrophoresis using low melting point agarose (Agarose L; manufactured by Nippon Gene Co., Ltd.), and a DNA fragment was recovered from a gel at the detected band part. About 100 ng of the recovered DNA fragment and about 1 µg of the aforementioned TATA DNA were mixed, and ligated with T4 ligase, thereby, a plasmid pGL3-TATA was prepared.

An oligonucleotide comprising a nucleotide sequence (SEQ ID NO: 17) upstream of a cholesterol 7α-hydroxylase (CYP7A1) gene of a human cytochrome P450 family containing a liver X receptor α-binding sequence (LXRE) and an oligonucleotide (SEQ ID NO: 18) comprising a nucleotide sequence complementary to the nucleotide sequence were synthesized, these were annealed to form a double-stranded DNA (hereinafter, referred to as LXRE DNA), and T4 ligase was acted on this to ligate LXRE DNA tandemly. On the resulting DNA fragment was acted T4 polynucleotide kinase to phosphorylate both ends thereof, thereby, a phosphorylated tandemly-ligated DNA fragment was obtained.

Then, pGL3-TATA was digested with a restriction enzyme Mlu I, and Escherichia coli-derived alkaline phosphatase was added, followed by incubation at 65°C for 1 hour. The incubated solution was subjected to low melting point agarose gel electrophoresis, and a DNA fragment was recovered from a gel at the detected band part. About 100 ng of the recovered DNA and about 1 µg of the aforementioned phosphorylated tandemly-ligated DNA fragment were mixed, T4 ligase was added thereto, and the mixture was ligated at 16°C for 30 minutes. The ligation was performed according to the method recommended by a kit used. Competent cells of Escherichia coli DH5α (manufactured by TAKARA SHUZO Co., Ltd.) were transformed by the resulting ligation reaction solution according to the method recommended by the manufacturer. The resulting bacterial cells were spread on a LB agar medium containing ampicillin on which X-gal and isopropyl β-D-galactopyranoside (IPTG) had been coated, and this was cultured at 37°C overnight. After culturing, a plasmid DNA was prepared from the ampicillin-resistant white colony formed on the agar medium. Further, a nucleotide sequence of the inserted DNA fragment was determined. From the resulting nucleotide sequence, a plasmid having a DNA fragment in which four LXRE DNAs are tandemly ligated to a MluI site of pGL3-TATA was selected, and this was named as plasmid pGL3-TATA-LXREx4.

### Example 9 (Assessment of function of liver X receptor α splicing mutant proteins by reporter assay in a transient expression system)

A human fetal kidney-derived HEK293 cell strain (ATCC; CRL-1573) was subcultured in a Dulbecco modified Eagle medium (DMEM) containing 10% bovine fetal serum (FBS) from which low-molecular substances had been removed with charcoal dextran, and penicillin (100 unit/ml) and streptomycin (100 µg/ml) as an antibiotic, and not containing phenolred at 37°C under the presence of 5% CO₂. The subculturedHEK293 cell strainwas seeded on a 10 cm cell culturing dish to about 2×10⁶ cells. This was cultured at 37°C for one day, and each 1 µg of a normal liver X receptor α and a human liver X receptor α splicing mutant protein-expression plasmid, and a reporter plasmid pGL3-TATA LXRE x4 were introduced into the cells using Effectene (manufactured by Qiagen) according to the method recommended by the kit. This was cultured at 37°C for 24 hours, and the cells were recovered by pipetting. The recovered cells were uniformly suspended, and this was seeded on a 96-well plate. Further, various compounds which had been dissolved with ethanol were added to the plate. This was further cultured at 37°C for about 2 days, 50 µl/well of a 5-fold diluted cytolytic agent PGC50 (manufactured by Nippon Gene Co., Ltd.) was added to the plate, and this was allowed to stand at room temperature for 30 minutes while sometimes gently shaken, to lyse cells. Each 10 µl of the thus prepared cell lysate was taken into a 96-well white sample plate (manufactured by Berthold Technologies), each 50 µl/well of an enzyme substrate solution PGL100 (manufactured by Nippon Gene Co., Ltd.) was added with a luminometer LB96p (manufactured by Berthold Technologies), and luminescence intensity was immediately measured for 5 seconds.

Results of measurement of various liver X receptor α ligands (agonists) on a normal liver X receptor α or liver X receptor α splicing mutant proteins were shown in Figs. 5 and 6, respectively.

### INDUSTRIAL APPLICABILITY

According to the present invention, it becomes possible to provide liver X receptor α splicing mutant proteins which are novel isoforms of liver X receptor α involved in inhibition of normal cholesterol metabolism by a normal liver X receptor α, genes thereof, and use of thereof.
Free Text in Sequence Listing
   SEQ ID NO: 9
Designed oligonucleotide primer for PCR
   SEQ ID NO: 10
Designed oligonucleotide primer for PCR
   SEQ ID NO: 11
Designed oligonucleotide primer for PCR
   SEQ ID NO: 12
Designed oligonucleotide primer for PCR
   SEQ ID NO: 13
Designed oligonucleotide primer for PCR
   SEQ ID NO: 14
Designed oligonucleotide primer for PCR
   SEQ ID NO: 15
Designed oligonucleotide to synthesize promoter DNA
   SEQ ID NO: 16
Designed oligonucleotide to synthesize promoter DNA
   SEQ ID NO: 17
Designed oligonucleotide to synthesize liver X receptor binding element
   SEQ ID NO: 18
Designed oligonucleotide to synthesize liver X receptor binding element

## Claims

1. A liver X receptor α splicing mutant protein which is an isoform of a liver X receptor α, and which comprises at least an amino acid sequence encoded by exon 5 of a liver X receptor α gene.

2. A liver X receptor α splicing mutant protein which is an isoform of a liver X receptor α, and which contains an amino acid sequence encoded by intron 5 of a liver X receptor α gene.

3. The liver X receptor α splicing mutant protein according to claim 2, which comprises any of the following amino acid sequences:
(1) the amino acid sequence of SEQ ID NO: 1,
(2) an amino acid sequence which is substantially identical to the amino acid sequence of SEQ ID NO: 1, and
(3) an amino acid sequence which has 95% ormore amino acid identity with the amino acid sequence of SEQ ID NO: 1.

4. A liver X receptor α splicing mutant protein which is an isoform of a liver X receptor α, and which contains an amino acid sequence encoded by a portion of intron 6 of a liver X receptor α gene.

5. The liver X receptor α splicing mutant protein according to claim 4, which comprises any of the following amino acid sequences:
(1) the amino acid sequence of SEQ ID NO: 2,
(2) an amino acid sequence which is substantially identical to the amino acid sequence of SEQ ID NO: 2, and
(3) an amino acid sequence which has 95% or more amino acid identity with the amino acid sequence of SEQ ID NO: 2.

6. The liver X receptor α splicing mutant protein according to any one of claims 1 to 5, which has weaker transcription activating ability as compared with ligand-dependent transcription activating ability of a normal liver X receptor α.

7. A polynucleotide comprising
a nucleotide sequence encoding an amino acid sequence of the liver X receptor α splicing mutant protein as defined in claim 3, or
a nucleotide sequence complementary to said nucleotide sequence.

8. A polynucleotide comprising
the nucleotide sequence of SEQ ID NO: 3, or
a nucleotide sequence complementary to said nucleotide sequence.

9. A polynucleotide comprising
a nucleotide sequence encoding an amino acid sequence of the liver X receptor α splicing mutant protein as defined in claim 5, or
a nucleotide sequence complementary to said nucleotide sequence.

10. A polynucleotide comprising
the nucleotide sequence of SEQ ID NO: 4, or
a nucleotide sequence complementary to said nucleotide sequence.

11. A polypeptide which is a partial polypeptide of the liver X receptor α splicing mutant protein as defined in claim 1, and which comprises an amino acid sequence that is not present in a normal liver X receptor α, within an amino acid sequence of the liver X receptor α splicing mutant protein as defined in claim 1.

12. The polypeptide according to claim 11, which comprises any of the following amino acid sequences:
(1) the amino acid sequence of SEQ ID NO: 5, and
(2) the amino acid sequence of SEQ ID NO: 6.

13. A polynucleotide comprising
a nucleotide sequence encoding an amino acid sequence of a polypeptide as defined in claim 12, or
a nucleotide sequence complementary to said nucleotide sequence.

14. A polynucleotide comprising
the nucleotide sequence of SEQ ID NO: 7, or
a nucleotide sequence complementary to said nucleotide sequence.

15. A polynucleotide comprising
the nucleotide sequence of SEQ ID NO: 8, or
a nucleotide sequence complementary to said nucleotide sequence.

16. A recombinant vector containing
a polynucleotide that comprises a nucleotide sequence encoding an amino acid sequence of the liver X receptor α splicing mutant protein as defined in any one of claims 1 to 6, or
a polynucleotide as defined in any one of claims 7 to 10.

17. A transformant in which
a polynucleotide comprising a nucleotide sequence encoding an amino acid sequence of the liver X receptor α splicing mutant protein as defined in any one of claims 1 to 6, or
a polynucleotide as defined in any one of claims 7 to 10 is introduced into a host cell.

18. A process for producing a vector comprising a step of introducing a polynucleotide that comprises a nucleotide sequence encoding an amino acid sequence of the liver X receptor α splicing mutant protein as defined in any one of claims 1 to 6, or a polynucleotide as defined in any one of claims 7 to 10, into a vector capable of autonomously replicating in a host cell.

19. A process for producing a liver X receptor α splicing mutant protein comprising a step of culturing a transformant as defined in claim 17 to produce a liver X receptor α splicing mutant protein.

20. A gene-deficient animal which is deficient in the ability to express a polynucleotide comprising a nucleotide sequence encoding an amino acid sequence of a liver X receptor α splicing mutant protein as defined in any one of claims 1 to 6, or a polynucleotide as defined in any one of claims 7 to 10.

21. A recombinant vector containing a polynucleotide that comprises a nucleotide sequence encoding an amino acid sequence of a polypeptide as defined in any one of claims 11 to 12, or a polynucleotide as defined in any one of claims 13 to 15.

22. A transformant in which a polynucleotidecomprising a nucleotide sequence encoding an amino acid sequence of a polypeptide as defined in any one of claims 11 to 12, or a polynucleotide as defined in any one of claims 13 to 15 is introduced into a host cell.

23. A method for screening a substance which acts on a liver X receptor α splicing mutant protein, comprising a step of contacting a test substance with a liver X receptor α splicing mutant protein, and a step of measuring a change in the liver X receptor α splicing mutant protein.

24. The screening method according to claim 23, wherein the liver X receptor α splicing mutant protein is a liver X receptor α splicing mutant protein as defined in any one of claims 1 to 6.

25. A method for screening a substance which acts on a liver X receptor α splicing mutant protein, comprising a step of contacting a test substance with a cell producing a liver X receptor α splicing mutant protein, a step of measuring an amount of production of other protein which undergoes expression regulation by the liver X receptor α splicing mutant protein in the cell, and a step of assessing the property of acting of the test substance on the liver X receptor α splicing mutant protein based on the amount of production.

26. The screening method according to claim 25, wherein the liver X receptor α splicing mutant protein is a liver X receptor α splicing mutant protein as defined in any one of claims 1 to 6.

27. A method for screening a substance which acts on a liver X receptor α splicing mutant protein, comprising a first step of contacting a cell producing a liver X receptor α splicing mutant protein with a ligand for the liver X receptor α splicing mutant protein, and measuring an amount of production of other protein which undergoes expression regulation by the liver X receptor α splicing mutant protein, a second step of contacting a cell producing the liver X receptor α splicing mutant protein with both of the ligand for the liver X receptor α splicing mutant protein and a test substance, and measuring an amount of production of other protein which undergoes expression regulation by the liver X receptor α splicing mutant protein, and a step of assessing of the properties of the test substance of antagonizing the ligand based on a difference in amounts of production of the aforementioned other proteins measured in the first step and the second step.

28. The screening method according to claim 27, wherein the liver X receptor α splicing mutant protein is a liver X receptor α splicing mutant protein as defined in any one of claims 1 to 6.

29. A substance which acts on a liver X receptor α splicing mutant protein, and which has been selected by the screening method as defined in any one of claims 23 to 28.

30. An antagonist to a substance as defined in claim 29, wherein said substance acts on a liver X receptor α splicing mutant protein.

31. An antibody which specifically recognizes a liver X receptor α splicing mutant protein as defined in any one of claims 1 to 6, or a part of the antibody.

32. A primer designed based on a polynucleotide as defined in any one of claims 7 to 10.

33. Use of a DNA probe specific for a nucleotide sequence of a polynucleotide as defined in any one of claims 13 to 15, for gene therapy.

34. Use of an RNA probe specific for a nucleotide sequence of a polynucleotide as defined in any one of claims 13 to 15, for gene therapy.

35. A method for detecting the presence or the absence of a liver X receptor α splicing mutant protein as defined in any one of claims 1 to 6, comprising a first step of synthesizing complementary DNA (cDNA) from messenger RNA (mRNA) in a biological sample, a second step of amplifying a portion of the complementary DNA (cDNA) corresponding to a polynucleotide that comprises a nucleotide sequence encoding an amino acid sequence of a liver X receptor α splicing mutant protein as defined in any one of claims 1 to 6 or a polypeptide as defined in any one of claims 11 to 12, or a polynucleotide as defined in any one of claims 13 to 15, and a third step of detecting the amplified portion.

36. The method according to claim 35, wherein the portion is amplified by the PCR method using, as a primer, one or a plurality of polynucleotides selected from polynucleotides comprising any of nucleotide sequences of SEQ ID NO: 9, SEQ ID NO: 10, SEQ ID NO: 11, SEQ ID NO: 12, SEQ ID NO: 13 and SEQ ID NO: 14 in a second step.

37. The method according to claim 35, wherein the polynucleotide that comprises a nucleotide sequence encoding an amino acid sequence of a liver X receptor α splicing mutant protein as defined in any one of claims 1 to 6 or a polypeptide as defined in any one of claims 11 to 12, or the polynucleotide as defined in any one of claims 13 to 15 is a polynucleotide comprising a nucleotide sequence of SEQ ID NO: 7 or 8.

38. A method for detecting the presence or the absence of a liver X receptor α splicing mutant protein as defined in any one of claims 1 to 6, comprising a step of detecting the liver X receptor α splicing mutant protein based on an antigen-antibody reaction using an antibody that can specifically detect a liver X receptor α splicing mutant protein as defined in any one of claims 1 to 6 or a polypeptide as defined in any one of claims 11 to 12.

39. A method for detecting the presence or the absence of a liver X receptor α splicing mutant protein as defined in any one of claims 1 to 6, comprising a first step of binding to a microtiter well a primary antibody that can specifically detect a liver X receptor α splicing mutant protein as defined in any one of claims 1 to 6 or a polypeptide as defined in any one of claims 11 to 12, a second step of binding to the primary antibody on the microtiter well a liver X receptor α splicing mutant protein as defined in any one of claims 1 to 6 in a biological sample, a third step of removing all biological samples which are in an unbound state in the microtiter well after the second step, a forth step of binding to the liver X receptor α splicing mutant protein bound to the primary antibody a labeled secondary antibody which can bind to an epitope different from those for the primary antibody in the liver X receptor α splicing mutant protein, a fifth step of removing all secondary antibodies which are in an unbound state in the microtiter well after the forth step, and a sixth step of detecting the presence or the absence of the secondary antibody using a label on the secondary antibody as an index after the fifth step.

40. Use of a liver X receptor α splicing mutant protein as a marker for investigating cholesterol metabolism abnormality.

41. The use according to claim 40, wherein the liver X receptor α splicing mutant protein is a liver X receptor α splicing mutant protein as defined in claim 2, 3, 4, 5 or 6, or a liver X receptor α splicing mutant protein which is an isoform of a liver X receptor α, and which is deficient in an amino acid sequence encoded by exon 5 of a liver X receptor α gene.

42. Use of a liver X receptor α splicing mutant protein which is an isoform of a liver X receptor α, and which is deficient in an amino acid sequence encoded by exon 5 of a liver X receptor α gene, as a marker for investigating the presence of a cancer cell.

43. Use of a polynucleotide comprising a nucleotide sequence encoding an amino acid sequence of a liver X receptor α splicing mutant protein, as a marker for investigating cholesterol metabolism abnormality.

44. The use according to claim 43, wherein the polynucleotide comprising a nucleotide sequence encoding an amino acid sequence of a liver X receptor α splicingmutant protein is a polynucleotide as defined in any one of claims 7 to 10 and 13 to 15.

45. Use of a polynucleotide comprising a nucleotide sequence encoding an amino acid sequence of a liver X receptor α splicing mutant protein, wherein said protein is an isoform of a liver X receptor α and is deficient in an amino acid sequence encoded by exon 5 of a liver X receptor α gene, as a marker for investigating the presence of a cancer cell.


SEQUENCE LISTING

<110> Sumitomo Chemical Company, Limited

<120> LIVER X RECEPTOR a SPLICING MUTANT PROTEIN, GENE THEREOF AND USE THEREOF

<130>

<150> JP 2003/135461
<151> 2003-05-14

<160> 18

<210> 1
<211> 511
<212> PRT
<213> Homo sapiens

<400> 1
Met Ser Leu Trp Leu Gly Ala Pro Val Pro Asp Ile Pro Pro Asp Ser
1               5                   10                  15
Ala Val Glu Leu Trp Lys Pro Gly Ala Gln Asp Ala Ser Ser Gln Ala
            20                  25                  30
Gln Gly Gly Ser Ser Cys Ile Leu Arg Glu Glu Ala Arg Met Pro His
        35                  40                  45
Ser Ala Gly Gly Thr Ala Gly Val Gly Leu Glu Ala Ala Glu Pro Thr
    50                  55                  60
Ala Leu Leu Thr Arg Ala Glu Pro Pro Ser Glu Pro Thr Glu Ile Arg
65                  70                  75                  80
Pro Gln Lys Arg Lys Lys Gly Pro Ala Pro Lys Met Leu Gly Asn Glu
                85                  90                  95
Leu Cys Ser Val Cys Gly Asp Lys Ala Ser Gly Phe His Tyr Asn Val
            100                 105                 110
Leu Ser Cys Glu Gly Cys Lys Gly Phe Phe Arg Arg Ser Val Ile Lys
        115                 120                 125
Gly Ala His Tyr Ile Cys His Ser Gly Gly His Cys Pro Met Asp Thr
    130                 135                 140
Tyr Met Arg Arg Lys Cys Gln Glu Cys Arg Leu Arg Lys Cys Arg Gln
145                 150                 155                 160
Ala Gly Met Arg Glu Glu Cys Val Leu Ser Glu Glu Gln Ile Arg Leu
                165                 170                 175
Lys Lys Leu Lys Arg Gln Glu Glu Glu Gln Ala His Ala Thr Ser Leu
            180                 185                 190
Pro Pro Arg Arg Ser Ser Pro Pro Gln Ile Leu Pro Gln Leu Ser Pro
        195                 200                 205
Glu Gln Leu Gly Met Ile Glu Lys Leu Val Ala Ala Gln Gln Gln Cys
    210                 215                 220
Asn Arg Arg Ser Phe Ser Asp Arg Leu Arg Val Thr Pro Trp Pro Met
225                 230                 235                 240
Ala Pro Asp Pro His Ser Arg Glu Ala Arg Gln Gln Arg Phe Ala His
                245                 250                 255
Phe Thr Glu Leu Ala Ile Val Ser Val Gln Glu Ile Val Asp Phe Ala
            260                 265                 270
Lys Gln Leu Pro Gly Phe Leu Gln Leu Ser Arg Glu Asp Gln Ile Ala
        275                 280                 285
Leu Leu Lys Thr Ser Ala Ile Glu Val Ala Gly Glu Gly Gln Gly Met
    290                 295                 300
Lys Gly Glu Ala Glu Trp Asp Tyr Leu Trp Glu Gly Pro Pro Asp Ile
305                 310                 315                 320
Glu Leu Gly Glu Pro Asn Leu Leu Gly Ser Arg Asp Glu Glu Asn Arg
                325                 330                 335
Pro Pro Trp Lys Arg Pro Cys Ser Lys Thr Ser Pro Pro Ser Pro Arg
            340                 345                 350
Leu Arg Phe Ala Ala Cys Val Gln Val Met Leu Leu Glu Thr Ser Arg
        355                 360                 365
Arg Tyr Asn Pro Gly Ser Glu Ser Ile Thr Phe Leu Lys Asp Phe Ser
    370                 375                 380
Tyr Asn Arg Glu Asp Phe Ala Lys Ala Gly Leu Gln Val Glu Phe Ile

385                 390                 395                 400
Asn Pro Ile Phe Glu Phe Ser Arg Ala Met Asn Glu Leu Gln Leu Asn
                405                 410                 415
Asp Ala Glu Phe Ala Leu Leu Ile Ala Ile Ser Ile Phe Ser Ala Asp
            420                 425                 430
Arg Pro Asn Val Gln Asp Gln Leu Gln Val Glu Arg Leu Gln His Thr
        435                 440                 445
Tyr Val Glu Ala Leu His Ala Tyr Val Ser Ile His His Pro His Asp
    450                 455                 460
Arg Leu Met Phe Pro Arg Met Leu Met Lys Leu Val Ser Leu Arg Thr
465                 470                 475                 480
Leu Ser Ser Val His Ser Glu Gln Val Phe Ala Leu Arg Leu Gln Asp
                485                 490                 495
Lys Lys Leu Pro Pro Leu Leu Ser Glu Ile Trp Asp Val His Glu
            500                 505                 510

<210> 2
<211> 356
<212> PRT
<213> Homo sapiens

<400> 2
Met Ser Leu Trp Leu Gly Ala Pro Val Pro Asp Ile Pro Pro Asp Ser
 1               5                  10                  15
Ala Val Glu Leu Trp Lys Pro Gly Ala Gln Asp Ala Ser Ser Gln Ala
            20                  25                  30
Gln Gly Gly Ser Ser Cys Ile Leu Arg Glu Glu Ala Arg Met Pro His
        35                  40                  45
Ser Ala Gly Gly Thr Ala Gly Val Gly Leu Glu Ala Ala Glu Pro Thr
    50                  55                  60
Ala Leu Leu Thr Arg Ala Glu Pro Pro Ser Glu Pro Thr Glu Ile Arg
65                  70                  75                  80
Pro Gln Lys Arg Lys Lys Gly Pro Ala Pro Lys Met Leu Gly Asn Glu
                85                  90                  95
Leu Cys Ser Val Cys Gly Asp Lys Ala Ser Gly Phe His Tyr Asn Val
            100                 105                 110
Leu Ser Cys Glu Gly Cys Lys Gly Phe Phe Arg Arg Ser Val Ile Lys
        115                 120                 125
Gly Ala His Tyr Ile Cys His Ser Gly Gly His Cys Pro Met Asp Thr
    130                 135                 140
Tyr Met Arg Arg Lys Cys Gln Glu Cys Arg Leu Arg Lys Cys Arg Gln
145                 150                 155                 160
Ala Gly Met Arg Glu Glu Cys Val Leu Ser Glu Glu Gln Ile Arg Leu
                165                 170                 175
Lys Lys Leu Lys Arg Gln Glu Glu Glu Gln Ala His Ala Thr Ser Leu
            180                 185                 190
Pro Pro Arg Arg Ser Ser Pro Pro Gln Ile Leu Pro Gln Leu Ser Pro
        195                 200                 205
Glu Gln Leu Gly Met Ile Glu Lys Leu Val Ala Ala Gln Gln Gln Cys
    210                 215                 220
Asn Arg Arg Ser Phe Ser Asp Arg Leu Arg Val Thr Pro Trp Pro Met
225                 230                 235                 240
Ala Pro Asp Pro His Ser Arg Glu Ala Arg Gln Gln Arg Phe Ala His
                245                 250                 255
Phe Thr Glu Leu Ala Ile Val Ser Val Gln Glu Ile Val Asp Phe Ala
            260                 265                 270
Lys Gln Leu Pro Gly Phe Leu Gln Leu Ser Arg Glu Asp Gln Ile Ala
        275                 280                 285
Leu Leu Lys Thr Ser Ala Ile Glu Val Met Leu Leu Glu Thr Ser Arg
    290                 295                 300
Arg Tyr Asn Pro Gly Ser Glu Ser Ile Thr Phe Leu Lys Asp Phe Ser
305                 310                 315                 320
Tyr Asn Arg Glu Asp Phe Ala Lys Ala Gly Ser Arg Ser Val Ala Gln
                325                 330                 335
Ala Glu Cys Ser Gly Val Ile Asn His Gly Ser Met Gln Pro Arg Pro
            340                 345                 350
Ser Gly Leu Lys
        355

<210> 3
<211> 1536
<212> DNA
<213> Homo sapiens

<400> 3
atgtccttgt ggctgggggc ccctgtgcct gacattcctc ctgactctgc ggtggagctg 60
tggaagccag gcgcacagga tgcaagcagc caggcccagg gaggcagcag ctgcatcctc 120
agagaggaag ccaggatgcc ccactctgct gggggtactg caggggtggg gctggaggct 180
gcagagccca cagccctgct caccagggca gagccccctt cagaacccac agagatccgt 240
ccacaaaagc ggaaaaaggg gccagccccc aaaatgctgg ggaacgagct atgcagcgtg 300
tgtggggaca aggcctcggg cttccactac aatgttctga gctgcgaggg ctgcaaggga 360
ttcttccgcc gcagcgtcat caagggagcg cactacatct gccacagtgg cggccactgc 420
cccatggaca cctacatgcg tcgcaagtgc caggagtgtc ggcttcgcaa atgccgtcag 480
gctggcatgc gggaggagtg tgtcctgtca gaagaacaga tccgcctgaa gaaactgaag 540
cggcaagagg aggaacaggc tcatgccaca tccttgcccc ccaggcgttc ctcacccccc 600
caaatcctgc cccagctcag cccggaacaa ctgggcatga tcgagaagct cgtcgctgcc 660
cagcaacagt gtaaccggcg ctccttttct gaccggcttc gagtcacgcc ttggcccatg 720
gcaccagatc cccatagccg ggaggcccgt cagcagcgct ttgcccactt cactgagctg 780
gccatcgtct ctgtgcagga gatagttgac tttgctaaac agctacccgg cttcctgcag 840
ctcagccggg aggaccagat tgccctgctg aagacctctg cgatcgaggt ggctggagaa 900
gggcaaggga tgaagggaga agcagagtgg gattatctgt gggaggggcc tccagacatc 960
gagctgggag agccaaatct gctgggaagc agggatgagg agaatcggcc tccctggaag 1020
aggccatgct ccaagaccag ccctcctagt ccccgtttga ggtttgctgc ttgtgtgcag 1080
gtgatgcttc tggagacatc tcggaggtac aaccctggga gtgagagtat caccttcctc 1140
aaggatttca gttataaccg ggaagacttt gccaaagcag ggctgcaagt ggaattcatc 1200
aaccccatct tcgagttctc cagggccatg aatgagctgc aactcaatga tgccgagttt 1260
gccttgctca ttgctatcag catcttctct gcagaccggc ccaacgtgca ggaccagctc 1320
caggtggaga ggctgcagca cacatatgtg gaagccctgc atgcctacgt ctccatccac 1380
catccccatg accgactgat gttcccacgg atgctaatga aactggtgag cctccggacc 1440
ctgagcagcg tccactcaga gcaagtgttt gcactgcgtc tgcaggacaa aaagctccca 1500
ccgctgctct ctgagatctg ggatgtgcac gaatga 1536

<210> 4
<211> 1453
<212> DNA
<213> Homo sapiens

<400> 4
atgtccttgt ggctgggggc ccctgtgcct gacattcctc ctgactctgc ggtggagctg 60
tggaagccag gcgcacagga tgcaagcagc caggcccagg gaggcagcag ctgcatcctc 120
agagaggaag ccaggatgcc ccactctgct gggggtactg caggggtggg gctggaggct 180
gcagagccca cagccctgct caccagggca gagccccctt cagaacccac agagatccgt 240
ccacaaaagc ggaaaaaggg gccagccccc aaaatgctgg ggaacgagct atgcagcgtg 300
tgtggggaca aggcctcggg cttccactac aatgttctga gctgcgaggg ctgcaaggga 360
ttcttccgcc gcagcgtcat caagggagcg cactacatct gccacagtgg cggccactgc 420
cccatggaca cctacatgcg tcgcaagtgc caggagtgtc ggcttcgcaa atgccgtcag 480
gctggcatgc gggaggagtg tgtcctgtca gaagaacaga tccgcctgaa gaaactgaag 540
cggcaagagg aggaacaggc tcatgccaca tccttgcccc ccaggcgttc ctcacccccc 600
caaatcctgc cccagctcag cccggaacaa ctgggcatga tcgagaagct cgtcgctgcc 660
cagcaacagt gtaaccggcg ctccttttct gaccggcttc gagtcacgcc ttggcccatg 720
gcaccagatc cccatagccg ggaggcccgt cagcagcgct ttgcccactt cactgagctg 780
gccatcgtct ctgtgcagga gatagttgac tttgctaaac agctacccgg cttcctgcag 840
ctcagccggg aggaccagat tgccctgctg aagacctctg cgatcgaggt gatgcttctg 900
gagacatctc ggaggtacaa ccctgggagt gagagtatca ccttcctcaa ggatttcagt 960
tataaccggg aagactttgc caaagcaggg tctcgctctg tcgcccaggc tgaatgcagt 1020
ggtgtgatca atcatggctc aatgcagcct cgaccttctg ggctcaagtg atcctccttg 1080
agtagctggg actacagggc tgcaagtgga attcatcaac cccatcttcg agttctccag 1140
ggccatgaat gagctgcaac tcaatgatgc cgagtttgcc ttgctcattg ctatcagcat 1200
cttctctgca gaccggccca acgtgcagga ccagctccag gtggagaggc tgcagcacac 1260
atatgtggaa gccctgcatg cctacgtctc catccaccat ccccatgacc gactgatgtt 1320
cccacggatg ctaatgaaac tggtgagcct ccggaccctg agcagcgtcc actcagagca 1380
agtgtttgca ctgcgtctgc aggacaaaaa gctcccaccg ctgctctctg agatctggga 1440
tgtgcacgaa tga 1453

<210> 5
<211> 64

<212> PRT
<213> Homo sapiens

<400> 5
Val Ala Gly Glu Gly Gln Gly Met Lys Gly Glu Ala Glu Trp Asp Tyr
1               5                   10                  15
Leu Trp Glu Gly Pro Pro Asp Ile Glu Leu Gly Glu Pro Asn Leu Leu
            20                  25                  30
Gly Ser Arg Asp Glu Glu Asn Arg Pro Pro Trp Lys Arg Pro Cys Ser
        35                  40                  45
Lys Thr Ser Pro Pro Ser Pro Arg Leu Arg Phe Ala Ala Cys Val Gln
    50                  55                  60

<210> 6
<211> 26
<212> PRT
<213> Homo sapiens

<400> 6
Ser Arg Ser Val Ala Gln Ala Glu Cys Ser Gly Val Ile Asn His Gly
1               5                   10                  15
Ser Met Gln Pro Arg Pro Ser Gly Leu Lys
            20                  25

<210> 7
<211> 192
<212> DNA
<213> Homo sapiens

<400> 7
gtggctggag aagggcaagg gatgaaggga gaagcagagt gggattatct gtgggagggg  60
cctccagaca tcgagctggg agagccaaat ctgctgggaa gcagggatga ggagaatcgg 120
cctccctgga agaggccatg ctccaagacc agccctccta gtccccgttt gaggtttgct 180
gcttgtgtgc ag                                                      192

<210> 8
<211> 109
<212> DNA
<213> Homo sapiens

<400> 8
ggtctcgctc tgtcgcccag gctgaatgca gtggtgtgat caatcatggc tcaatgcagc  60
ctcgaccttc tgggctcaag tgatcctcct tgagtagctg ggactacag              109

<210> 9
<211> 23
<212> DNA
<213> Artificial sequence

<220>
<223> Designed oligonucleotide primer for PCR

<400> 9
gccccagctc agcccggaac aac          23

<210> 10
<211> 24
<212> DNA
<213> Artificial sequence

<220>
<223> Designed oligonucleotide primer for PCR

<400> 10
gtctgcagag aagatgctga tagc    24

<210> 11
<211> 34

<212> DNA
<213> Artificial sequence

<220>
<223> Designed oligonucleotide primer for PCR

<400> 11
cgaagcttgc caccatgtcc ttgtggctgg gggc          34

<210> 12
<211> 25
<212> DNA
<213> Artificial sequence

<220>
<223> Designed oligonucleotide primer for PCR

<400> 12
cgggatcctt cgtgcacatc ccaga          25

<210> 13
<211> 25
<212> DNA
<213> Artificial sequence
<220>
<223> Designed oligonucleotide primer for PCR

<400> 13
ctggtcttgg agcatggcct cttcc          25

<210> 14
<211> 25
<212> DNA
<213> Artificial sequence
<220>
<223> Designed oligonucleotide primer for PCR

<400> 14
catgattgat cacaccactg cattc          25

<210> 15
<211> 52
<212> DNA
<213> Artificial sequence

<220>
<223> Designed oligonucleotide to synthesize promoter DNA

<400> 15
gatctcgact ataaagaggg caggctgtcc tctaagcgtc accacgactt ca          52

<210> 16
<211> 52
<212> DNA
<213> Artificial sequence

<220>
<223> Designed oligonucleotide to synthesize promoter DNA

<400> 16
agcttgaagt cgtggtgacg cttagaggac agcctgccct ctttatagtc ga          52

<210> 17
<211> 46
<212> DNA
<213> Artificial sequence

<220>

<223> Designed oligonucleotide to synthesize liver X receptor binding element

<400> 17
CGCGTTTGCT TTGGTCACTC AAGTTCAAGT TATTGA     46

<210> 18
<211> 46
<212> DNA
<213> Artificial sequence

<220>
<223> Designed oligonucleotide to synthesize liver X receptor binding element

<400> 18
cgcgtcaata acttgaactt gagtgaccaa agcaaa     46

SEQUENCE LISTING

<110> Sumitomo Chemical Company, Limited

<120> LIVER X RECEPTOR α SPLICING MUTANT PROTEIN, GENE THEREOF AND USE
THEREOF

<130>

<150> JP 2003/135461
<151> 2003-05-14

<160> 18

<210> 1
<211> 511
<212> PRT
<213> Homo sapiens

<400> 1
Met Ser Leu Trp Leu Gly Ala Pro Val Pro Asp Ile Pro Pro Asp Ser
1               5                   10                  15
Ala Val Glu Leu Trp Lys Pro Gly Ala Gln Asp Ala Ser Ser Gln Ala
            20                  25                  30
Gln Gly Gly Ser Ser Cys Ile Leu Arg Glu Glu Ala Arg Met Pro His
        35                  40                  45
Ser Ala Gly Gly Thr Ala Gly Val Gly Leu Glu Ala Ala Glu Pro Thr
    50                  55                  60

Ala Leu Leu Thr Arg Ala Glu Pro Pro Ser Glu Pro Thr Glu Ile Arg
65                  70                  75                  80
Pro Gln Lys Arg Lys Lys Gly Pro Ala Pro Lys Met Leu Gly Asn Glu
                85                  90                  95
Leu Cys Ser Val Cys Gly Asp Lys Ala Ser Gly Phe His Tyr Asn Val
            100                 105                 110
Leu Ser Cys Glu Gly Cys Lys Gly Phe Phe Arg Arg Ser Val Ile Lys
        115                 120                 125
Gly Ala His Tyr Ile Cys His Ser Gly Gly His Cys Pro Met Asp Thr
    130                 135                 140
Tyr Met Arg Arg Lys Cys Gln Glu Cys Arg Leu Arg Lys Cys Arg Gln
145                 150                 155                 160
Ala Gly Met Arg Glu Glu Cys Val Leu Ser Glu Glu Gln Ile Arg Leu
                165                 170                 175
Lys Lys Leu Lys Arg Gln Glu Glu Glu Gln Ala His Ala Thr Ser Leu
            180                 185                 190
Pro Pro Arg Arg Ser Ser Pro Pro Gln Ile Leu Pro Gln Leu Ser Pro
        195                 200                 205
Glu Gln Leu Gly Met Ile Glu Lys Leu Val Ala Ala Gln Gln Gln Cys
    210                 215                 220
Asn Arg Arg Ser Phe Ser Asp Arg Leu Arg Val Thr Pro Trp Pro Met
225                 230                 235                 240
Ala Pro Asp Pro His Ser Arg Glu Ala Arg Gln Gln Arg Phe Ala His
                245                 250                 255
Phe Thr Glu Leu Ala Ile Val Ser Val Gln Glu Ile Val Asp Phe Ala
            260                 265                 270
Lys Gln Leu Pro Gly Phe Leu Gln Leu Ser Arg Glu Asp Gln Ile Ala
        275                 280                 285

Leu Leu Lys Thr Ser Ala Ile Glu Val Ala Gly Glu Gly Gln Gly Met
    290                 295                 300
Lys Gly Glu Ala Glu Trp Asp Tyr Leu Trp Glu Gly Pro Pro Asp Ile
305                 310                 315                 320
Glu Leu Gly Glu Pro Asn Leu Leu Gly Ser Arg Asp Glu Glu Asn Arg
                325                 330                 335
Pro Pro Trp Lys Arg Pro Cys Ser Lys Thr Ser Pro Pro Ser Pro Arg
            340                 345                 350
Leu Arg Phe Ala Ala Cys Val Gln Val Met Leu Leu Glu Thr Ser Arg
        355                 360                 365
Arg Tyr Asn Pro Gly Ser Glu Ser Ile Thr Phe Leu Lys Asp Phe Ser
    370                 375                 380
Tyr Asn Arg Glu Asp Phe Ala Lys Ala Gly Leu Gln Val Glu Phe Ile
385                 390                 395                 400
Asn Pro Ile Phe Glu Phe Ser Arg Ala Met Asn Glu Leu Gln Leu Asn
                405                 410                 415
Asp Ala Glu Phe Ala Leu Leu Ile Ala Ile Ser Ile Phe Ser Ala Asp
            420                 425                 430
Arg Pro Asn Val Gln Asp Gln Leu Gln Val Glu Arg Leu Gln His Thr
        435                 440                 445
Tyr Val Glu Ala Leu His Ala Tyr Val Ser Ile His His Pro His Asp
    450                 455                 460
Arg Leu Met Phe Pro Arg Met Leu Met Lys Leu Val Ser Leu Arg Thr
465                 470                 475                 480
Leu Ser Ser Val His Ser Glu Gln Val Phe Ala Leu Arg Leu Gln Asp
                485                 490                 495
Lys Lys Leu Pro Pro Leu Leu Ser Glu Ile Trp Asp Val His Glu
            500                 505                 510

<210> 2

<211> 356

<212> PRT

<213> Homo sapiens

<400> 2

Met Ser Leu Trp Leu Gly Ala Pro Val Pro Asp Ile Pro Pro Asp Ser
1               5                   10                  15

Ala Val Glu Leu Trp Lys Pro Gly Ala Gln Asp Ala Ser Ser Gln Ala
            20                  25                  30

Gln Gly Gly Ser Ser Cys Ile Leu Arg Glu Glu Ala Arg Met Pro His
        35                  40                  45

Ser Ala Gly Gly Thr Ala Gly Val Gly Leu Glu Ala Ala Glu Pro Thr
    50                  55                  60

Ala Leu Leu Thr Arg Ala Glu Pro Pro Ser Glu Pro Thr Glu Ile Arg
65                  70                  75                  80

Pro Gln Lys Arg Lys Lys Gly Pro Ala Pro Lys Met Leu Gly Asn Glu
                85                  90                  95

Leu Cys Ser Val Cys Gly Asp Lys Ala Ser Gly Phe His Tyr Asn Val
            100                 105                 110

Leu Ser Cys Glu Gly Cys Lys Gly Phe Phe Arg Arg Ser Val Ile Lys
        115                 120                 125

Gly Ala His Tyr Ile Cys His Ser Gly Gly His Cys Pro Met Asp Thr
    130                 135                 140

Tyr Met Arg Arg Lys Cys Gln Glu Cys Arg Leu Arg Lys Cys Arg Gln
145                 150                 155                 160

Ala Gly Met Arg Glu Glu Cys Val Leu Ser Glu Glu Gln Ile Arg Leu

                165                 170                 175
Lys Lys Leu Lys Arg Gln Glu Glu Glu Gln Ala His Ala Thr Ser Leu
            180                 185                 190
Pro Pro Arg Arg Ser Ser Pro Pro Gln Ile Leu Pro Gln Leu Ser Pro
        195                 200                 205
Glu Gln Leu Gly Met Ile Glu Lys Leu Val Ala Ala Gln Gln Gln Cys
    210                 215                 220
Asn Arg Arg Ser Phe Ser Asp Arg Leu Arg Val Thr Pro Trp Pro Met
225                 230                 235                 240
Ala Pro Asp Pro His Ser Arg Glu Ala Arg Gln Gln Arg Phe Ala His
                245                 250                 255
Phe Thr Glu Leu Ala Ile Val Ser Val Gln Glu Ile Val Asp Phe Ala
            260                 265                 270
Lys Gln Leu Pro Gly Phe Leu Gln Leu Ser Arg Glu Asp Gln Ile Ala
        275                 280                 285
Leu Leu Lys Thr Ser Ala Ile Glu Val Met Leu Leu Glu Thr Ser Arg
    290                 295                 300
Arg Tyr Asn Pro Gly Ser Glu Ser Ile Thr Phe Leu Lys Asp Phe Ser
305                 310                 315                 320
Tyr Asn Arg Glu Asp Phe Ala Lys Ala Gly Ser Arg Ser Val Ala Gln
                325                 330                 335
Ala Glu Cys Ser Gly Val Ile Asn His Gly Ser Met Gln Pro Arg Pro
            340                 345                 350
Ser Gly Leu Lys
        355

<210> 3
<211> 1536

<212> DNA
<213> Homo sapiens

<400> 3
atgtccttgt ggctgggggc ccctgtgcct gacattcctc ctgactctgc ggtggagctg 60
tggaagccag gcgcacagga tgcaagcagc caggcccagg gaggcagcag ctgcatcctc 120
agagaggaag ccaggatgcc ccactctgct gggggtactg caggggtggg gctggaggct 180
gcagagccca cagccctgct caccagggca gagccccctt cagaacccac agagatccgt 240
ccacaaaagc ggaaaaaggg gccagccccc aaaatgctgg ggaacgagct atgcagcgtg 300
tgtggggaca aggcctcggg cttccactac aatgttctga gctgcgaggg ctgcaaggga 360
ttcttccgcc gcagcgtcat caagggagcg cactacatct gccacagtgg cggccactgc 420
cccatggaca cctacatgcg tcgcaagtgc caggagtgtc ggcttcgcaa atgccgtcag 480
gctggcatgc gggaggagtg tgtcctgtca gaagaacaga tccgcctgaa gaaactgaag 540
cggcaagagg aggaacaggc tcatgccaca tccttgcccc ccaggcgttc ctcacccccc 600
caaatcctgc cccagctcag cccggaacaa ctgggcatga tcgagaagct cgtcgctgcc 660
cagcaacagt gtaaccggcg ctccttttct gaccggcttc gagtcacgcc ttggcccatg 720
gcaccagatc cccatagccg ggaggcccgt cagcagcgct ttgcccactt cactgagctg 780
gccatcgtct ctgtgcagga gatagttgac tttgctaaac agctacccgg cttcctgcag 840
ctcagccggg aggaccagat tgccctgctg aagacctctg cgatcgaggt ggctggagaa 900
gggcaaggga tgaagggaga agcagagtgg gattatctgt gggaggggcc tccagacatc 960
gagctgggag agccaaatct gctgggaagc agggatgagg agaatcggcc tccctggaag 1020
aggccatgct ccaagaccag ccctcctagt ccccgtttga ggtttgctgc ttgtgtgcag 1080
gtgatgcttc tggagacatc tcggaggtac aaccctggga gtgagagtat caccttcctc 1140
aaggatttca gttataaccg ggaagacttt gccaaagcag ggctgcaagt ggaattcatc 1200
aaccccatct tcgagttctc cagggccatg aatgagctgc aactcaatga tgccgagttt 1260
gccttgctca ttgctatcag catcttctct gcagaccggc ccaacgtgca ggaccagctc 1320
caggtggaga ggctgcagca cacatatgtg gaagccctgc atgcctacgt ctccatccac 1380
catccccatg accgactgat gttcccacgg atgctaatga aactggtgag cctccggacc 1440

ctgagcagcg tccactcaga gcaagtgttt gcactgcgtc tgcaggacaa aaagctccca 1500
ccgctgctct ctgagatctg ggatgtgcac gaatga 1536

<210> 4
<211> 1453
<212> DNA
<213> Homo sapiens

<400> 4
atgtccttgt ggctgggggc ccctgtgcct gacattcctc ctgactctgc ggtggagctg 60
tggaagccag gcgcacagga tgcaagcagc caggcccagg gaggcagcag ctgcatcctc 120
agagaggaag ccaggatgcc ccactctgct gggggtactg caggggtggg gctggaggct 180
gcagagccca cagccctgct caccagggca gagccccctt cagaacccac agagatccgt 240
ccacaaaagc ggaaaaaggg gccagccccc aaaatgctgg ggaacgagct atgcagcgtg 300
tgtggggaca aggcctcggg cttccactac aatgttctga gctgcgaggg ctgcaaggga 360
ttcttccgcc gcagcgtcat caagggagcg cactacatct gccacagtgg cggccactgc 420
cccatggaca cctacatgcg tcgcaagtgc caggagtgtc ggcttcgcaa atgccgtcag 480
gctggcatgc gggaggagtg tgtcctgtca gaagaacaga tccgcctgaa gaaactgaag 540
cggcaagagg aggaacaggc tcatgccaca tccttgcccc ccaggcgttc ctcacccccc 600
caaatcctgc cccagctcag cccggaacaa ctgggcatga tcgagaagct cgtcgctgcc 660
cagcaacagt gtaaccggcg ctccttttct gaccggcttc gagtcacgcc ttggcccatg 720
gcaccagatc cccatagccg ggaggcccgt cagcagcgct ttgcccactt cactgagctg 780
gccatcgtct ctgtgcagga gatagttgac tttgctaaac agctacccgg cttcctgcag 840
ctcagccggg aggaccagat tgccctgctg aagacctctg cgatcgaggt gatgcttctg 900
gagacatctc ggaggtacaa ccctgggagt gagagtatca ccttcctcaa ggatttcagt 960
tataaccggg aagactttgc caaagcaggg tctcgctctg tcgcccaggc tgaatgcagt 1020
ggtgtgatca atcatggctc aatgcagcct cgaccttctg ggctcaagtg atcctccttg 1080
agtagctggg actacagggc tgcaagtgga attcatcaac cccatcttcg agttctccag 1140

ggccatgaat gagctgcaac tcaatgatgc cgagtttgcc ttgctcattg ctatcagcat 1200
cttctctgca gaccggccca acgtgcagga ccagctccag gtggagaggc tgcagcacac 1260
atatgtggaa gccctgcatg cctacgtctc catccaccat ccccatgacc gactgatgtt 1320
cccacggatg ctaatgaaac tggtgagcct ccggaccctg agcagcgtcc actcagagca 1380
agtgtttgca ctgcgtctgc aggacaaaaa gctcccaccg ctgctctctg agatctggga 1440
tgtgcacgaa tga 1453

<210> 5
<211> 64
<212> PRT
<213> Homo sapiens

<400> 5
Val Ala Gly Glu Gly Gln Gly Met Lys Gly Glu Ala Glu Trp Asp Tyr
1               5                   10                  15
Leu Trp Glu Gly Pro Pro Asp Ile Glu Leu Gly Glu Pro Asn Leu Leu
            20                  25                  30
Gly Ser Arg Asp Glu Glu Asn Arg Pro Pro Trp Lys Arg Pro Cys Ser
        35                  40                  45
Lys Thr Ser Pro Pro Ser Pro Arg Leu Arg Phe Ala Ala Cys Val Gln
    50                  55                  60

<210> 6
<211> 26
<212> PRT
<213> Homo sapiens

<400> 6

Ser Arg Ser Val Ala Gln Ala Glu Cys Ser Gly Val Ile Asn His Gly
1               5                   10                  15
Ser Met Gln Pro Arg Pro Ser Gly Leu Lys
                20                  25

<210> 7
<211> 192
<212> DNA
<213> Homo sapiens

<400> 7
gtggctggag aagggcaagg gatgaaggga gaagcagagt gggattatct gtgggagggg  60
cctccagaca tcgagctggg agagccaaat ctgctgggaa gcagggatga ggagaatcgg 120
cctccctgga agaggccatg ctccaagacc agccctccta gtccccgttt gaggtttgct 180
gcttgtgtgc ag                                                      192

<210> 8
<211> 109
<212> DNA
<213> Homo sapiens

<400> 8
ggtctcgctc tgtcgcccag gctgaatgca gtggtgtgat caatcatggc tcaatgcagc  60
ctcgaccttc tgggctcaag tgatcctcct tgagtagctg ggactacag             109

<210> 9
<211> 23
<212> DNA

<213> Artificial sequence

<220>
<223> Designed oligonucleotide primer for PCR

<400> 9
gccccagctc agcccggaac aac          23

<210> 10
<211> 24
<212> DNA
<213> Artificial sequence

<220>
<223> Designed oligonucleotide primer for PCR

<400> 10
gtctgcagag aagatgctga tagc     24

<210> 11
<211> 34
<212> DNA
<213> Artificial sequence

<220>
<223> Designed oligonucleotide primer for PCR

<400> 11

cgaagcttgc caccatgtcc ttgtggctgg gggc 34

<210> 12
<211> 25
<212> DNA
<213> Artificial sequence

<220>
<223> Designed oligonucleotide primer for PCR

<400> 12
cgggatcctt cgtgcacatc ccaga 25

<210> 13
<211> 25
<212> DNA
<213> Artificial sequence
<220>
<223> Designed oligonucleotide primer for PCR

<400> 13
ctggtcttgg agcatggcct cttcc 25

<210> 14
<211> 25
<212> DNA
<213> Artificial sequence
<220>

<223> Designed oligonucleotide primer for PCR

<400> 14
catgattgat cacaccactg cattc          25

<210> 15
<211> 52
<212> DNA
<213> Artificial sequence

<220>
<223> Designed oligonucleotide to synthesize promoter DNA

<400> 15
gatctcgact ataaagaggg caggctgtcc tctaagcgtc accacgactt ca          52

<210> 16
<211> 52
<212> DNA
<213> Artificial sequence

<220>
<223> Designed oligonucleotide to synthesize promoter DNA

<400> 16
agcttgaagt cgtggtgacg cttagaggac agcctgccct ctttatagtc ga          52

<210> 17

<211> 46

<212> DNA

<213> Artificial sequence

<220>

<223> Designed oligonucleotide to synthesize liver X receptor binding

element

<400> 17

CGCGTTTGCT TTGGTCACTC AAGTTCAAGT TATTGA      46

<210> 18

<211> 46

<212> DNA

<213> Artificial sequence

<220>

<223> Designed oligonucleotide to synthesize liver X receptor binding

element

<400> 18

cgcgtcaata acttgaactt gagtgaccaa agcaaa      46